# EUROPEAN PATENT SPECIFICATION

(11) **EP 2 659 885 B1**
(45) Date of publication and mention of the grant of the patent: **09.11.2016**
(21) Application number: 13178966.1
(22) Date of filing: 04.08.2008
(51) Int. Cl.: A61K 31/155, A61K 31/167, A61K 31/18, A61K 31/341, A61K 31/36, A61K 31/381, A61K 31/415, A61K 31/435, A61P 31/14, A61K 31/7064, A61K 31/7068, A61K 45/06, A61K 31/7056, A61K 38/21, A61P 1/16, A61P 31/12

(54) **Hepatitis C Antiviral Compositions and Methods**
Antivirale Hepatitis-C-Zusammensetzungen und Verfahren
Compositions antivirales contre l'hépatite C et procédés

(30) Priority: 03.08.2007 AU 2007904154 P
(43) Date of publication of application: 06.11.2013
(62) Divisional of application: 08782880.2
(73) Proprietor: Biotron Limited, Sydney, New South Wales 2000 (AU)
(72) Inventor: Ewart, Gary Dinneen, Hackett, Australian Capital Territory 2602 (AU); Luscombe, Carolyn Anne, Pueblo, CO Colorado 81001 (US); Miller, Michelle, Tarramurra, New South Wales 2074 (AU)
(74) Representative: Clements, Andrew Russell Niel

(56) References cited:
- WO-A1-2006/135978
- WO-A2-2004/112687
- WO-A2-2006/127360
- ZEKRY A ET AL: "Treatment of acute hepatitis C infection: more pieces of the puzzle?", JOURNAL OF HEPATOLOGY, ELSEVIER, vol. 42, no. 3, 1 March 2005 (2005-03-01), pages 293-296, XP027780405, ISSN: 0168-8278 [retrieved on 2005-03-01]
- LUSCOMBE, C. A. ET AL.: GLOBAL ANTIVIRAL JOURNAL, vol. 3, no. supp. 2, ABS. 65, December 2007 (2007-12), pages 69-70, XP002687182,
- BIOTRON: "BIT225 HIGHLY SYNERGISTIC WITH CURRENT ANTI-HEPATITIS C VIRUS THERAPIESm", 20070807, 7 August 2007 (2007-08-07), pages 1-2, XP007921292,

## Description

### FIELD OF INVENTION

The present invention relates to novel compositions having activity against Hepatitis C virus (HCV). The invention also relates to methods for retarding, reducing or otherwise inhibiting HCV growth and/or functional activity.

### BACKGROUND OF THE INVENTION

Any discussion of the prior art throughout the specification should in no way be considered as an admission that such prior art is widely known or forms part of common general knowledge in the field.

Currently, there is a great need for the development of new treatments that are effective against viral infections, particularly against viral infections which are associated with high morbidity and mortality, and which impact on sizable populations, for example Hepatitis C virus (HCV). Treatments that are currently available are inadequate or ineffective in large proportions of patients infected with HCV.

Hepatitis C is a blood-borne, infectious, viral disease that is caused by a hepatotropic virus called HCV. The infection can cause liver inflammation that is often asymptomatic, but ensuing chronic hepatitis can result later in cirrhosis (fibrotic scarring of the liver) and liver cancer. HCV is one of six known hepatitis viruses: A, B, C, D, E, G and is spread by blood-to-blood contact with an infected person's blood. The symptoms can be medically managed, and a proportion of patients can be cleared of the virus by a long course of anti-viral medicines. Although early medical intervention is helpful, people with HCV infection often experience mild symptoms, and consequently do not seek treatment. An estimated 150-200 million people worldwide are infected with HCV. Those with a history of intravenous drug use, inhaled drug usage, tattoos, or who have been exposed to blood via unsafe sex are at increased risk of contracting this disease. Hepatitis C is the leading cause of liver transplant in the United States.

Hepatitis C presents as two distinct clinical stages. Firstly, Hepatitis C presents as acute Hepatitis C, which refers to the first 6 months after infection with HCV. Between 60% to 70% of people infected develop no symptoms during the acute phase. In the minority of patients who experience acute phase symptoms, they are generally mild and nonspecific, and rarely lead to a specific diagnosis of Hepatitis C. Symptoms of acute Hepatitis C infection include decreased appetite, fatigue, abdominal pain, jaundice, itching, and flu-like symptoms.

HCV is usually detectable in the blood within one to three weeks after infection, and antibodies to the virus are generally detectable within 3 to 12 weeks. Approximately 20-30% of persons infected with HCV clear the virus from their bodies during the acute phase as shown by normalization in liver function tests (LFTs) such as alanine transaminase (ALT) and aspartate transaminase (AST) normalization, as well as plasma HCV-RNA clearance (this is known as spontaneous viral clearance). The remaining 70-80% of patients infected with HCV develop chronic Hepatitis C.

Chronic Hepatitis C is defined as infection with HCV persisting for more than six months. Clinically, it is often asymptomatic (without jaundice) and it is mostly discovered accidentally.

The natural course of chronic Hepatitis C varies considerably from person to person. Virtually all people infected with HCV have evidence of inflammation on liver biopsy. However, the rate of progression of liver scarring (fibrosis) shows significant variability among individuals. Recent data suggests that among untreated patients, roughly one-third progress to liver cirrhosis in less than 20 years. Another third progress to cirrhosis within 30 years. The remainder of patients appear to progress so slowly that they are unlikely to develop cirrhosis within their lifetimes. Factors that have been reported to influence the rate of HCV disease progression include age, gender, alcohol consumption, HIV coinfection and a fatty liver.

Symptoms specifically suggestive of liver disease are typically absent until substantial scarring of the liver has occurred. However, Hepatitis C is a systemic disease and patients may experience a wide spectrum of clinical manifestations ranging from an absence of symptoms to a more symptomatic illness prior to the development of advanced liver disease. Generalized signs and symptoms associated with chronic Hepatitis C include fatigue, marked weight loss, flu-like symptoms, muscle pain, joint pain, intermittent low-grade fevers, itching, sleep disturbances, abdominal pain, appetite changes, nausea, diarrhea, dyspepsia, cognitive changes, depression, headaches, and mood swings.

Once chronic Hepatitis C has progressed to cirrhosis, signs and symptoms may appear that are generally caused by either decreased liver function or increased pressure in the liver circulation, a condition known as portal hypertension. Possible signs and symptoms of liver cirrhosis include ascites, a tendency to bruise and bleed, bone pain, varices, fatty stools (steatorrhea), jaundice, and a syndrome of cognitive impairment known as hepatic encephalopathy.

The diagnosis of Hepatitis C is rarely made during the acute phase of the disease because the majority of people infected experience no symptoms during this phase. Those who do experience acute phase symptoms are rarely ill enough to seek medical attention. The diagnosis of chronic Hepatitis C is also challenging due to the absence or lack of specific symptoms until advanced liver disease develops, which may not occur until decades into the disease.

Current treatment ("standard of care") is a combination of pegylated interferon alpha and the antiviral drug Ribavirin for a period of 24 or 48 weeks, depending on the virus genotype. Further, the efficacy of this combination therapy, in its various forms, also depends on the virus genotype and ranges from 14% to 82%.

WO 2006/135978A1 discloses the use of the same class of acylguanidine compounds optionally with antiviral agents for the treatment of HCV infections. The specific compounds BIT-225 and BIT-314 are mentioned in this document. WO 2004/112687A2 discloses the use of guanidine derivatives, inter alia naphtoylguanidine derivatives as antiviral compounds optionally with antiviral agents for the treatment of HCV. A. Zekry et al., Journal of Hepatology 2005, vol 42, nr. 3, 293-296 and WO 2006/127360 A2 both report the use of interferon/peg interferon as standard treatment of Hepatitis C Virus. C.A. Luscombe et al., Global Antiviral Journal 2007, vol. 3, no. suppl. 2, Abstract 65, 69-70 discloses the synergistic use of BIT225 in combination with recombinant alpha -2b INF (rIFNα-2b) and Ribavirin in an in vitro model system for HCV.

To improve the prospect of treating and preventing viral infections, and to deal with ongoing viral evolution, there is an on-going need to identify molecules capable of inhibiting various aspects of the viral life cycle. Accordingly, there is a need for additional novel compositions and agents with antiviral activity.

It is an object of the present invention to overcome or ameliorate at least one of the disadvantages of the prior art, or to provide a useful alternative.

### SUMMARY OF THE INVENTION

The present invention is concerned with certain compositions, preferably synergistic compositions, comprising novel antiviral compounds, useful in the treatment of HCV infection, that fall under the classification of substituted acylguanidines. More particularly the present invention is concerned with synergistic compositions comprising one or more substituted acylguanidines and one or more known antiviral compounds.

According to a first aspect, the present invention provides a composition for the treatment of HCV, comprising:
(A) 5-(1-methylpyrazol-4-yl)2-naphthoylguanidine or (6-(1-methylpyrazol-4-yl)-2-nahpthoyl)guanidine,
   or a pharmaceutically acceptable salt thereof, and
(B) IFN α-2b,
   wherein:
   5-(1-methylpyrazol-4-yl)2-naphthoylguanidine has the structure 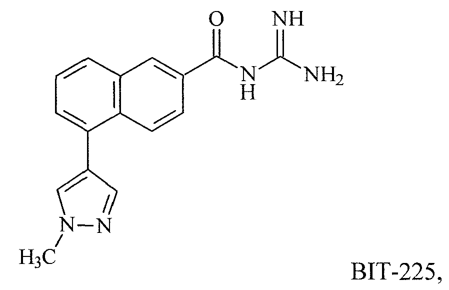 and
   (6-(1-methylpyrazol-4-yl)-2-naphthoyl)guanidine has the structure

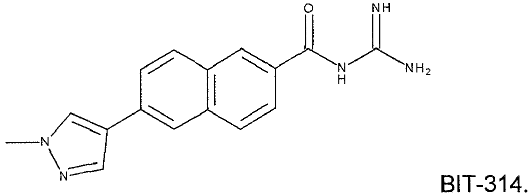

Advantageously, the compositions in accordance with the present invention are synergistic compositions wherein the effect of the compound and the at least one additional antiviral agent is greater than the sum of the effects of the compound and at least one additional antiviral agent alone.

According to a second aspect, the present invention provides a pharmaceutical composition for use in the treatment of HCV, comprising a composition according to the first aspect and one or more pharmaceutical acceptable carriers or excipients.

According to a third aspect, there is provided a composition as defined according to the first aspect for use in the treatment of HCV invention as a combined preparation for simultaneous, separate or sequential use, in any order.

The individual components of the composition may be administered separately in a sequential manner and in any order.

Compositions and formulations of the present invention may be administered in any manner, including but not limited to, intravenously (iv), intraperitoneally, subcutaneously, intracranially, intradermally, intramuscularly, intraocularly, intrathecally, intracerebrally, intranasally, transmucosally, or by infusion orally, rectally, via iv drip, patch or implant. The compositions may be in the form of powder, tablet, capsule, liquid, suspension or other similar dosage form.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to".

### BRIEF DESCRIPTION OF THE DRAWINGS

**Figure 1** graphically shows inhibition of GBV-B replication by BIT 225 and BIT100;
**Figure 2** graphically shows a dose response curve of various concentrations of BIT 225 against BVDV;
**Figure 3** graphically shows a dose response curve of various concentrations of IFN against BVDV;
**Figure 4** graphically shows a dose response curve of various concentrations of Ribavirin against BVDV;
**Figure 5** graphically shows the levels of virus inhibition seen with 31nM BIT225 and/or 1.25µg Ribavirin in the presence of absence of IFNα, and
**Figure 6** shows the full-range dose response curves for BIT225 in the presence of 5 and 10 IU/m IFNα and shows the enhanced antiviral effect by addition of 1.25 µg/ml. The inset shows the full range dose response curves for Ribavirin in the presence of 5 and 10 IU/m IFNα.
**Figure 7** shows individual dose response curves for 2'-C-methyladenosine and 2'-C-methylcytidine against BVDV.
**Figures 8** and **9** show the changes to dose response curves for BIT225 in the presence of various concentrations of 2'-C-methyladenosine or 2'-C-methylcytidine, respectively.
**Figure 10** shows full-range dose response curves for BIT314 in the presence of and various concentrations of rIFNα-2b.
**Figure 11** illustrates the enhanced antiviral effect by addition of 5 IU/m IFNα + 1.25 µg/ml ribavirin and 5 IU/m IFNα + 2.5 µg/ml ribavirin.

### DETAILED DESCRIPTION OF THE INVENTION

The present invention concerns compositions for the treatment of HCV comprising 5-(1-methylpyrazol-4-yl)2-naphthoylguanidine or (6-(1-methylpyrazol-4-yl)-2-nahpthoyl)guanidine,
or a pharmaceutically acceptable salt thereof, in combination with IFN α-2b, wherein:
5-(1-methylpyrazol-4-yl)2-naphthoylguanidine has the structure 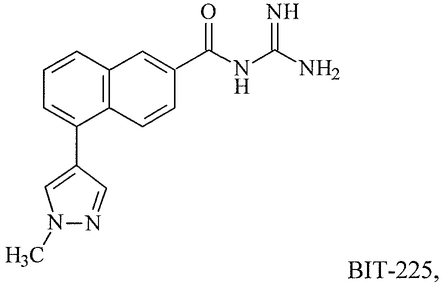 and
(6-(1-methylpyrazol-4-yl)-2-naphthoyl)guanidine has the structure

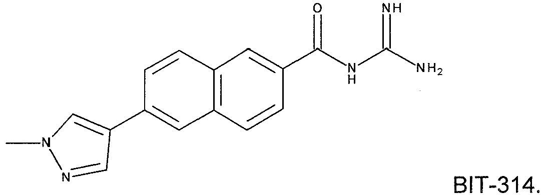

Particularly useful compounds for use in compositions of the present invention may be selected from the following compounds BIT-225and BIT-314. Further compounds disclosed herein also follow:
(3-benzoyl)cinnamoylguanidine comprising the structure 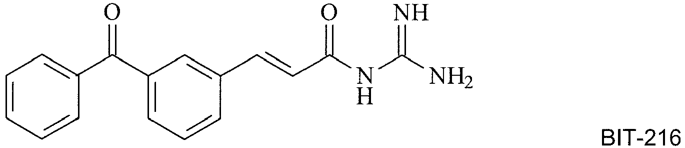
2,3-methylenedioxycinnamoyl guanidine comprising the structure 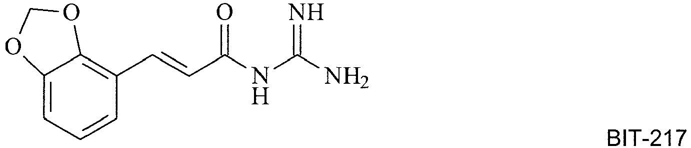
5-methyl-2-napthoylguanidine comprising the structure 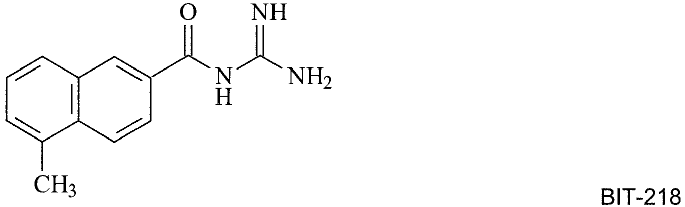
3(indan-4-yl)-propenoylguanidine comprising the structure 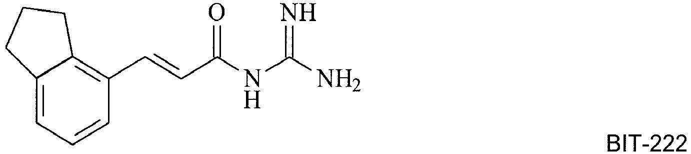
5-bromo-6-methoxy-2-napthoylguanidine comprising the structure 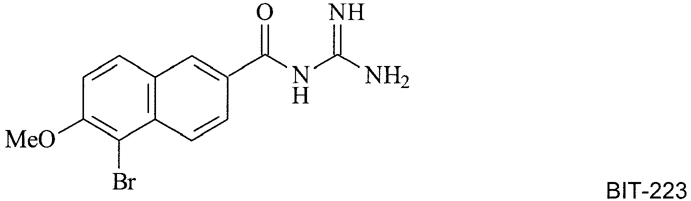
5-thiophen-3-yl-2-naphthoylguanidine comprising the structure 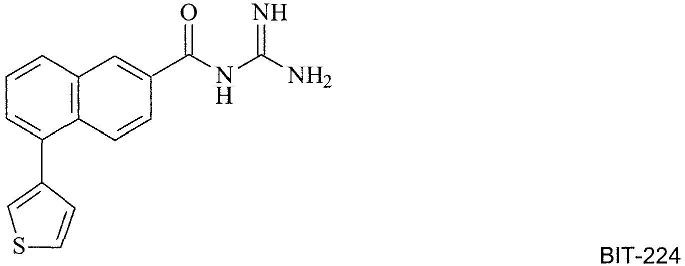
5-(1-methylpyrazol-4-yl)2-naphthoylguanidine comprising the structure 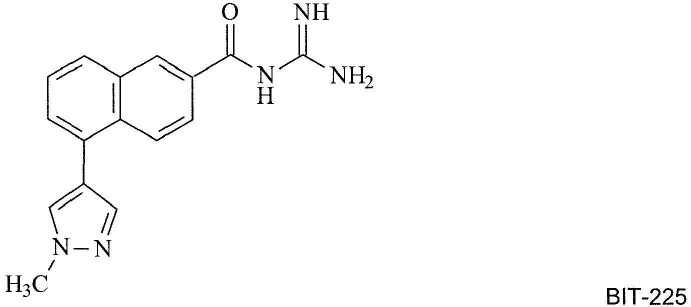
(1-methoxy-2-napthoyl)guanidine comprising the structure 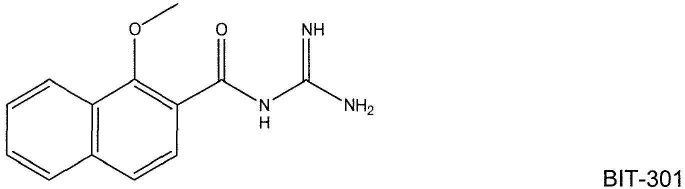
(3-methoxy-2-napthoyl)guanidine comprising the structure 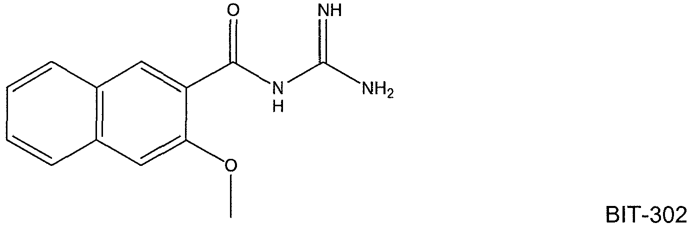
(5-bromo-2-napthoyl)guanidine comprising the structure 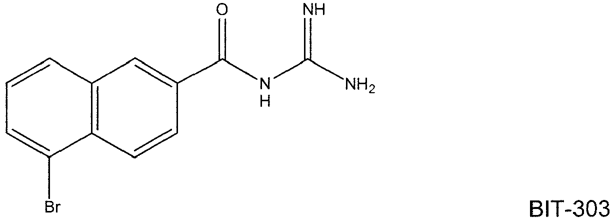
(1,4-dimethoxy--2-napthoyl)guanidine comprising the structure 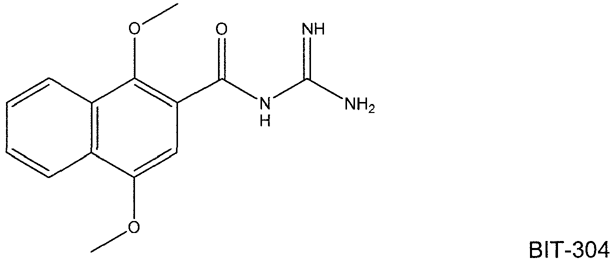
(6-(3-thienyl)-2-napthoyl)guanidine comprising the structure 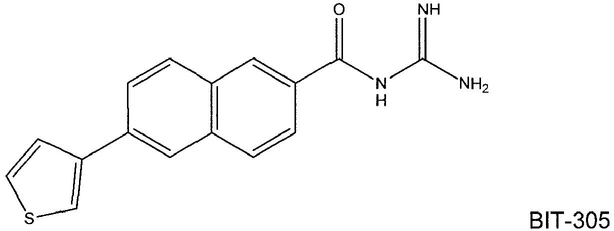
(6-methyl-2-napthoyl)guanidine comprising the structure 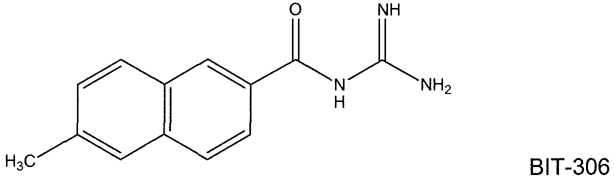 (5-phenyl-2-napthoyl)guanidine comprising the structure 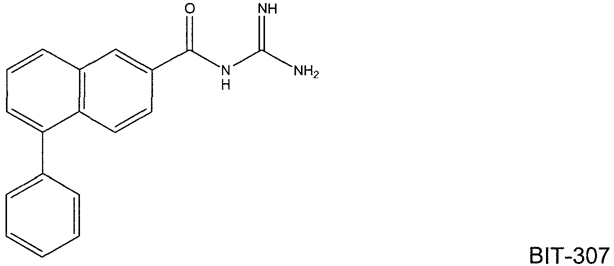
(5-(thien-2-yl)-2-napthoyl)guanidine comprising the structure 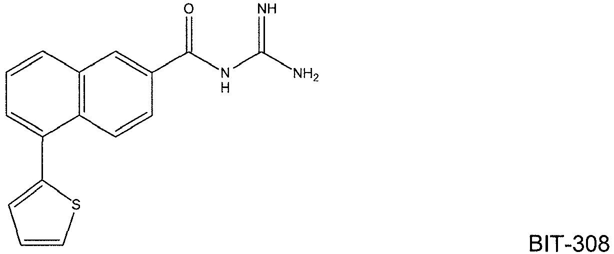
(5-(1-isobutyl-1H-pyrazol-4-yl)-2-napthoyl)guanidine comprising the structure 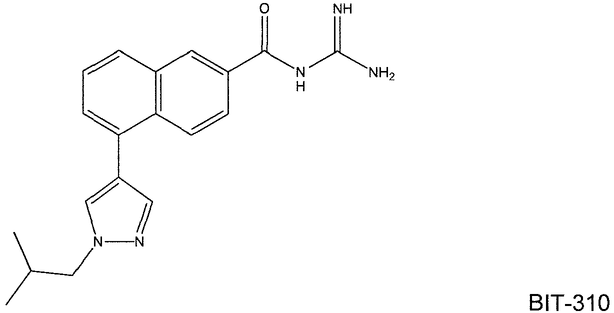
(5-(3-furyl)-2-napthoyl)guanidine comprising the structure 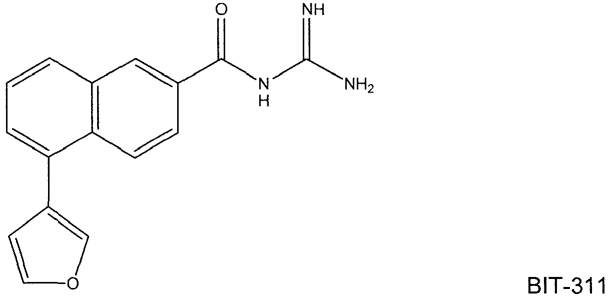
(5-cyclopropyl-2-napthoyl)guanidine 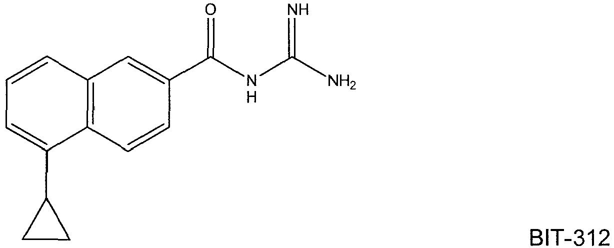
(5-chloro-2-napthoyl)guanidine 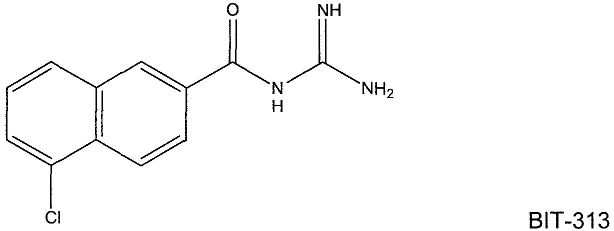
(6-(1-methylpryazol-4-yl)-2-napthoyl)guanidinium acetate 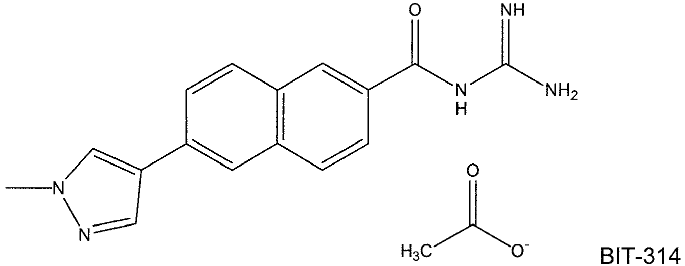
(5-(2,6-dimethoxypryridin-3-yl)-2-napthoyl)guanidine 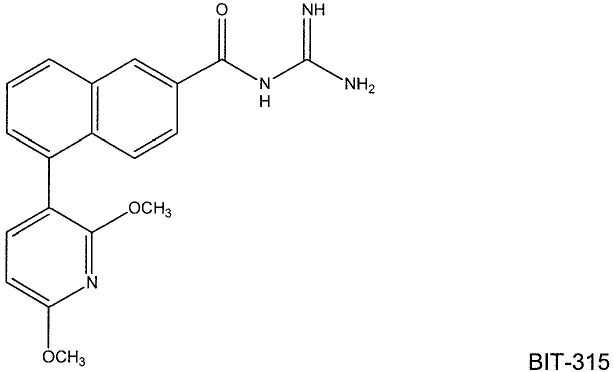
(5-(2-chlorophenyl)-2-napthoyl)guanidine 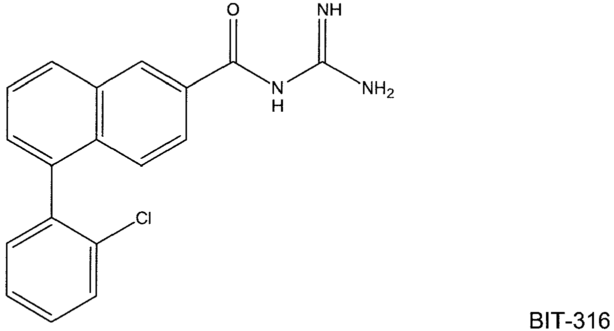
(5-(4-(acetylamino)phenyl)-2-napthoyl)guanidine

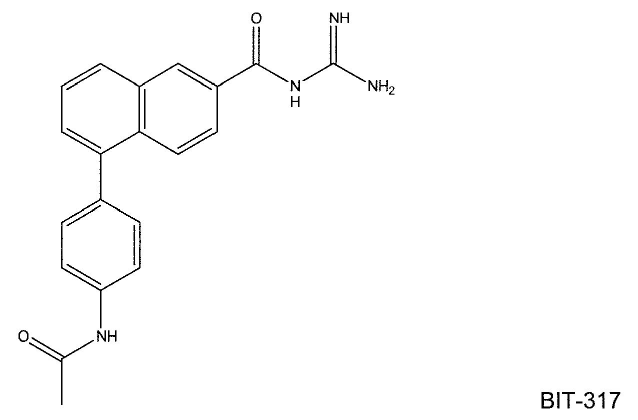

(5-(3-(acetylamino)phenyl)-2-napthoyl)guanidine 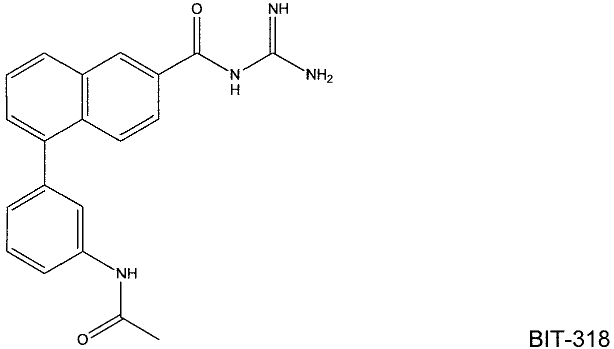
(5-(4-((methylsulphonyl)amino)phenyl)-2-napthoyl)guanidine 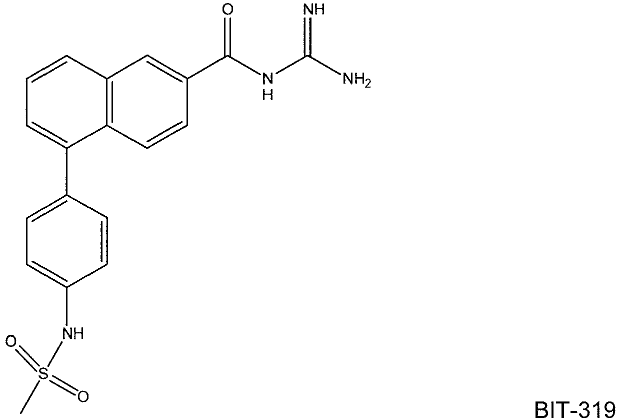
and pharmaceutically acceptable salts thereof. The amine or imine groups of the guanidyl portion of the compounds, disclosed herein can be present in any conventional form used for the provision of such compounds. For example, they maybe present as the free base, a hydrate, an organic or inorganic salt or combinations thereof.

The methods developed for screening the compounds of the present invention for antiviral activity are described in detail in PCT/AU2004/000866.

Reference to "HCV" should be understood as a reference to any hepatitis C virus strain, including homologues and mutants.

Reference to the "functional activity" of HCV should be understood as a reference to any one or more of the functions that HCV performs or is involved in.

Reference to the " viral replication" should be understood to include any one or more stages or aspects of the HCV life cycle, such as inhibiting the assembly or release of virions. Accordingly, the method of the present invention encompasses the mediation of HCV replication via the induction of a cascade of steps which lead to the mediation of any one or more aspects or stages of the HCV life cycle.

Reference to a "cell" infected with HCV should be understood as a reference to any cell, prokaryotic or eukaryotic, which has been infected with HCV. This includes, for example, immortal or primary cell lines, bacterial cultures and cells *in situ.*

It will be understood by those skilled in the art that the compounds of the invention may be administered in the form of a composition or formulation comprising pharmaceutically acceptable carriers and/or excipients.

The compositions described herein that comprise the compounds of the present invention, may include in combination one or more additional antiviral agents of any type, for example, a non-nucleoside HCV RNA-dependent RNA polymerase (RdRP) inhibitor, a nucleoside HCV RNA-dependent RNA polymerase (RdRP) inhibitor, a non-nucleoside HCV RNA protease inhibitor, a nucleoside HCV RNA protease inhibitor, non-nucleoside reverse transcriptase inhibitors (NNRTIs), a nucleoside reverse transcriptase inhibitor, a viral entry inhibitor, interferon, PEG-interferon, ribavirin and combinations thereof. It will be understood that the nucleoside and non-nucleoside inhibitors include analogs of nucleoside and non- nucleoside molecules. The polymerase inhibitors can target HCV NS5B and NS5A; the protease inhibitors can target HCV NS3 andNS4.

Nonlimiting examples of nucleoside analogue inhibitors of NS5B that may be used in combination therapies and in the compositions disclosed herein include valopicitabine, a prodrug of nucleoside analog 2^{>}-C-methylcytosine; JTK103; R04048; R-1479/R-1626, nucleoside analog of 4'- azidocytosine and prodrug thereof; and R-7128. Nonlimiting examples of non-nucleoside analog inhibitors (NNRTI) that maybe used in the compositions of the present invention include HCV-796, abenzofuran HCV polymerase inhibitor; GL60667 or "667"; and XTL-2125. Nonlimiting examples of serine protease inhibitors of NS3/4A of HCV that may be used in the compositions described herein include VX-950 ; SCH-503034; ACH-806/GS-9132; and BILN-2061 and ITMN-191.

Preferably, the at least one additional agent having anti-viral activity is an Interferon (IFN). Still more preferably, the Interferon is selected from the group consisting of type I and type II IFNs. Still more preferably the IFN is selected from the group consisting of IFNα, IFNβ and IFNγ. Still more preferably, the IFN is selected from the group consisting of, IFN α-2a, IFN α-2b, IFNα-n3, IFNα con-1, IFNβ-1a, IFN-β1, IFN-γ1b, peginterferon α-2b and peginterferon α-2a. Alternatively, the at least one additional agent having anti-viral activity may comprise one or more of IFNα-2b and Ribavirin; IFNα-2a and Ribavirin; pegylated IFNα-2a and Ribavirin or pegylated IFNα-2a and Ribavirin.

The at least one additional agent having anti-viral activity may comprise one or more compounds selected from a HCV protease inhibitor, a HCV polymerase inhibitor or a HCV serine protease inhibitor. Alternatively, the at least one additional agent having anti-viral activity may comprise one or more compounds selected from a monoclonal antibody, a botanical extract, a NS5A inhibitor, an immunomodulator, a thiazolide, an anti-phospholipid therapy, an antisense compound, an isatoribine, a broad spectrum immune stimulator, an inflammation/fibrosis inhibitor, a replicase inhibitor, a cyclophilin inhibitor, an imino sugar inhibitor, a pancaspase inhibitor or a polyclonal antibody.

Further, the at least one additional agent having anti-viral activity may comprise one or more anti-viral nucleoside analogues such as for example 2'-C-methyl nucleoside analogs. These may be selected from for example 2'-C-methyladenosine or 2'-C-methylcytidine.

The at least one additional agent having anti-viral activity may also comprise a vaccine selected from a therapeutic vaccine or a DNA based vaccine.

For a combination therapy in which the compounds of the present invention is used in conjunction with one or more conventional antiviral compounds or HCV antagonist agents, the compounds maybe provided to the subject prior to, subsequent to, or concurrently with the one or more conventional antiviral compounds or agents.

Preferably, the composition of the present invention is a synergistic composition, wherein the effect of the compound and the at least one additional agent having anti-viral activity is greater than the sum of the effects of the compound and at least one additional agent having anti-viral activity alone. Of course it will be understood that simple, additive, combinations of novel compounds and existing antiviral agents are also contemplated.

The subject of the viral inhibition is a mammal, such as, but not limited to, a human, a primate, a livestock animal, for example, a sheep, a cow, a horse, a donkey or a pig; a companion animal for example a dog or a cat; a laboratory test animal, for example, a mouse, a rabbit, a rat, a guinea pig or a hamster; or a captive wild animal, for example, a fox or a deer. Preferably, the subject is a primate. Most preferably, the subject is a human.

The present invention is particularly useful in the treatment and prophylaxis of a HCV infection. For example, in subjects infected with HCV, the antiviral activity may be affected in order to prevent replication of HCV thereby preventing the onset of acute or chronic Hepatitis C. Alternatively, the method of the present invention may be used to reduce serum HCV load or to alleviate HCV infection symptoms.

The present invention may be particularly useful either in the early stages of HCV infection to prevent the establishment of a HCV reservoir in affected cells or as a prophylactic treatment to be applied immediately prior to or for a period after exposure to a possible source of HCV.

Reference herein to "therapeutic" and "prophylactic" is to be considered in their broadest contexts. The term "therapeutic" does not necessarily imply that a mammal is treated until total recovery. Similarly, "prophylactic" does not necessarily mean that the subject will not eventually contract a disease condition. Accordingly, therapy and prophylaxis include amelioration of the symptoms of a particular condition or preventing or otherwise reducing the risk of developing a particular condition. The term "prophylaxis" may be considered as reducing the severity of onset of a particular condition. Therapy may also reduce the severity of an existing condition or the frequency of acute attacks.

In accordance with the present invention, more than one composition may be co-administered with one or more other therapeutic agents. By "co-administered" is meant simultaneous administration in the same formulation or in two different formulations via the same or different routes or sequential administration by the same or different routes. By "sequential" administration is meant a time difference of from seconds, minutes, hours or days between the administration of one compound and the next. The composition and the additional therapeutic agents may be administered in any order.

Routes of administration include, but are not limited to, intravenous (iv), intraperitoneal, subcutaneous, intracranial, intradermal, intramuscular, intraocular, intrathecal, intracerebral, intranasal, transmucosal, or by infusion orally, rectally, via iv drip, patch and implant. Intravenous routes are particularly preferred.

The present invention also extends to forms suitable for topical application such as creams, lotions and gels.

In a further embodiment, present invention provides a formulation for pulmonary or nasal administration for the treatment of HCV comprising a composition in accordance with the first aspect of the invention.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved and (b) the limitations inherent in the art of compounding.

Procedures for the preparation of dosage unit forms and topical preparations are readily available to those skilled in the art from texts such as Pharmaceutical Handbook. A Martindale Companion Volume Ed. Ainley Wade Nineteenth Edition The Pharmaceutical Press London*,* CRC Handbook of Chemistry and Physics Ed. Robert C. Weast Ph D. CRC Press Inc.*;* Goodman and Gilman's; The Pharmacological basis of Therapeutics. Ninth Ed. McGraw Hill*; Remington; and* The Science and Practice of Pharmacy. Nineteenth Ed. Ed. Alfonso R. Gennaro Mack Publishing Co. Easton Pennsylvania*.*

Effective amounts contemplated by the present invention will vary depending on the severity of the condition and the health and age of the recipient. In general terms, effective amounts may vary from 0.01 ng/kg body weight to about 100 mg/kg body weight.

The present invention will now be described in more detail with reference to specific but non-limiting examples describing synthetic protocols, viral inhibition and other anti-viral properties of the compounds of the present invention. Synthesis and screening for compounds that have antiviral activity can be achieved by the range of methodologies described herein or described in more detail in PCT/AU2004/000866.

It is to be understood, however, that the detailed description of specific procedures, compounds and methods is included solely for the purpose of exemplifying the present invention.

### EXAMPLES

Anti-viral activity of all the compounds of the present invention can be, and has been, ascertained using the methods described herein or described in detail in PCT/AU2004/000866. Further, methods for synthesis of the compounds of the invention, both generic and specific, described herein, described in referenced publications or otherwise known to those skilled in the art, can be used to prepare all the compounds of the present invention. Useful synthetic protocols are also provided in PCT/AU2006/000880.

More specifically, acylguanidines can be synthesised by a variety of methods including reacting guanidine (generally generated *in situ* from its hydrochloride salt) with a suitably activated derivative of a carboxylic acid. Examples include:
i) synthesis from acid chlorides, exemplified by Yamamoto et al., Chem. Pharm. Bull., 1997, 45, 1282
ii) synthesis from simple esters, exemplified by US patent 2,734,904. iii)synthesis from carboxylic acids, *via in situ* activation by carbonyldiimidazole, exemplified by US patent 5,883,133

The carboxylic acid precursors required for the preparation of the acylguanidines described herein were obtained by a variety of diverse methods. A large number of the substituted cinnamic acids are commercially available. In addition, numerous procedures for the synthesis of substituted cinnamic acids and their simple esters are well described in the art, including:
i) The reaction of malonic acid with an aromatic aldehyde and base (the Doebner Condensation), described in Chemical Reviews, 1944, 35, 156, and references contained therein.
ii) The reaction of acetic anhydride with an aromatic aldehyde and base (the Perkin Reaction), described in Organic Reactions, 1942, 1, 210, and references contained therein.
iii)The reaction of acrylic acid and simple esters thereof with an aromatic halide or aromatic triflate using palladium catalyst (the Heck Reaction), described in Organic Reactions, 1982, 28, 345, and references contained therein.
iv)The reaction of a trialkyl phosphonoacetate with an aromatic aldehyde and base (the Horner-Emmons Reaction), described in Organic Reactions, 1977, 25, 73, and references contained therein.

A number of simple halo, hydroxy, and alkoxy substituted naphthoic acids are either commercially available or known in the art and these provided the starting materials for the susbstituted naphthoylguanidines.

Naphthoic acids which are substituted with alkyl, cycloalkyl, aryl, and heterocyclic groups can often be prepared by reacting a halonaphthoic acid with a suitable organometallic reagent using a transition metal catalyst. One such variant of this methodology which was used to prepare a number of the substituted naphthoic acids used as precursors to the naphthoylguanidines described herein, was the palladium-catalyzed carbon-carbon bond forming reaction between bromonaphthoic acids and a suitably substituted boronic acid (or boronate ester) which is widely known in the art as the Suzuki coupling (described in Chemical Reviews, 1995, 95, 2457 and references therein). The reaction has wide applicability and can be used on a range of substituted halonaphthalenes which can then be further elaborated to introduce or unmask the required carboxylic acid group.

### 1. General Synthetic Methodology

### 1.1 General Procedure A - Preparation Of Aryl Triflates

To a solution of the phenol (10mmol) in pyridine (7mL) at 0°C was slowly added trifluoromethanesulphonic anhydride (11mmol, 1.leq). The resulting mixture was stirred at 0°C for a further 5 minutes before being allowed to warm to room temperature and stirred until TLC analysis showed that the starting phenol had been consumed. The mixture was then poured into water and extracted with ethyl acetate (x3). The combined extracts were washed sequentially with water, 1M aqueous hydrochloric acid, water and brine, then dried (MgSO₄) and concentrated *in vacuo* to give the crude product. The crude products were chromatographed over silica gel. Elution with a mixture of ethyl acetate/hexanes gave the desired aryl triflates, generally as colourless oils.

### 1.2 General Procedure B - Cinnamate Esters Via Heck Reaction Of Triflates

A mixture of the phenyl triflate (10mmol), methyl acrylate (14mmol, 1.4eq), triethylamine (40mmol, 4eq) and dichlorobis(triphenylphosphine)palladium (0.3mmol, 0.03eq) in dimethylformamide (30mL) was heated at 90°C. The reaction was monitored by GC/MS and fresh batches of methyl acrylate (1eq), triethylamine (2eq) and the palladium catalyst (0.03eq) were added as required, in an effort to force the reaction to completion. The mixture was then poured into water and extracted with a 1:1 mixture of diethyl ether/hexanes (x3). The combined extracts were washed with water, then brine, dried (MgSO₄), filtered through a pad of silica gel and the filtrate was concentrated *in vacuo* to give the crude product as an oil. The crude products were chromatographed over silica gel. Elution with a mixture of ethyl acetate/hexanes gave the desired methyl cinnamates, generally as colourless oils.

### 1.3 General Procedure C - Cinnamate Esters Via Heck Reaction Of Bromides

The aryl bromide (10mmol), palladium acetate (0.1mmol, 0.01eq) and tri-o-tolylphosphine (0.4mmol, 0.04eq) was added to the reaction flask and purged with nitrogen. To this, methyl acrylate (12.5mmol, 1.25eq), triethylamine (12.5mmol, 1.25eq) and dimethylformamide (1mL) were then added and the mixture was heated at 100°C. The reaction was monitored by GC/MS and fresh batches of palladium acetate (0.01eq), tri-o-tolylphosphine (0.04eq), methyl acrylate (1.25eq) and triethylamine (1.25eq) were added as required, in an effort to force the reaction to completion. The mixture was poured into water and extracted with a 1:1 mixture of diethyl ether/hexanes (x4). The combined extracts were washed with water, then brine, dried (MgSO₄), filtered through a pad of silica gel and the filtrate was concentrated *in vacuo* to give the crude product. The crude products were chromatographed over silica gel. Elution with a mixture of ethyl acetate/hexanes gave the desired methyl cinnamates, generally as colourless oils.

### 1.4 General Procedure D - Cinnamate Esters Via Horner-Emmons Reaction

A solution of triethyl phosphonoacetate (13mmol, 1.3eq) in anhydrous tetrahydrofuran (10mL) was added, over 5 minutes, to a suspension of sodium hydride (14.3mmol, 1.4eq) in anhydrous tetrahydrofuran (10mL) at 0°C under nitrogen. The mixture was then stirred at 0°C for 20 minutes. A solution of the benzaldehyde (10mmol) in tetrahydrofuran (15mL) was then added over 10 minutes at 0°C. The mixture was stirred at 0°C for a further 30 minutes before being allowed to stir at room temperature until GC/MS or TLC analysis showed that the benzaldehyde starting material had been consumed. Typically, reactions were allowed to stir at room temperature overnight to ensure complete consumption of the starting aldehyde. The mixture was poured into water, the organic layer was separated and the aqueous layer was extracted with ethyl acetate (x3). The combined organic extracts were then washed with water, then brine, dried (MgSO4) and concentrated *in vacuo* to give the crude product. The crude products were chromatographed over silica gel. Elution with a mixture of ethyl acetate/hexanes gave the desired ethyl cinnamates, generally as colourless oils.

### 1.5 General Procedure E - Preparation Of 5-Phenylpenta-2,4-Dienoic Esters

A solution of triethyl 4-phosphonocrotonate (26mmol, 1.3eq) in anhydrous tetrahydrofuran (10mL) was added, over 5 minutes, to a suspension of sodium hydride (28mmol, 1.4 eq, 60% suspension in oil) in anhydrous tetrahydrofuran (15mL) at 0°C under nitrogen. The mixture was then stirred at 0°C for 20 minutes. A solution of the benzaldehyde (20mmol) in tetrahydrofuran (10mL) was then added over 10 minutes at 0°C. The mixture was stirred at 0°C for a further 30 minutes and then it was allowed to stir at room temperature until GC/MS analysis showed that the starting aldehyde had been consumed. The reaction mixture was poured into water, the organic layer was separated and the aqueous layer was extracted with ethyl acetate (x3). The combined organic extracts were then washed with water, then brine, dried (MgSO₄) and concentrated *in vacuo* to give the crude ethyl ester as an oil. The crude products were chromatographed over silica gel. Elution with a mixture of ethyl acetate/hexanes gave the desired ethyl esters as colourless oils.

### 1.6 General Procedure F - Hydrolysis Of Esters

A solution of the ester (10mmol) in methanol (50mL) and water (5mL) was treated with an aqueous solution of 6M potassium hydroxide (20mmol, 2eq) and the mixture was heated under reflux until TLC analysis showed that no more starting material was present (usually 2-3 hours). The mixture was then poured into water (50-200mL) and acidified with concentrated hydrochloric acid to approximately pH 2. The resulting carboxylic acid was collected by filtration, washed with water and dried overnight under high vacuum.

### 1.7 General Procedure G - Suzuki Reactions Of Bromonaphthoic Acids

The bromo-2-naphthoic acid (2mmol), the appropriate boronic acid (or boronate ester) (2.2mmol), tetrakis(triphenylphosphine)palladium(0) (0.1mmol), and solid sodium carbonate (6.8mmol) were added to the reaction flask which was then purged with nitrogen. Acetonitrile (6mL) and water (2.5mL) were added and the mixture was heated under reflux with vigorous stirring until the starting bromo-2-naphthoic acid had been consumed. The reaction mixture was then partitioned between toluene (50mL) and 0.5M sodium hydroxide solution (100mL). The aqueous layer was washed with toluene (to remove any triphenylphosphine, 3 x 20mL) then acidified to pH 1 with concentrated hydrochloric acid. The naphthoic acid derivatives were extracted into ethyl acetate (4 x 20mL). The combined ethyl acetate extracts were washed with water (3 x 20mL) and brine (10mL), then dried (MgSO₄), filtered, and concentrated. The residue was analyzed by ¹H NMR, and chromatographed over silica gel (if required).

### 1.8 General Procedure H - Preparation Of Acylguanidines

To a suspension/solution of carboxylic acid (10mmol, 1.0eq) in dichloromethane (30mL) containing a drop of dimethylformamide was added oxalyl chloride (12mmol, 1.2eq) which caused the solution to effervesce. After stirring for 2 h, the resulting solution was evaporated to dryness under reduced pressure. The residue was dissolved in dry tetrahydrofuran (30mL) and added to a solution of guanidine hydrochloride (50mmol, 5.0eq) in 2M aqueous sodium hydroxide (30mL). The reaction was stirred at room temperature for 1h and then the tetrahydrofuran layer was separated. The aqueous layer was extracted with chloroform (100mL) followed by ethyl acetate (100mL) and the combined organic layers evaporated under reduced pressure. The resulting residue was partitioned between chloroform (200mL) and 2M aqueous sodium hydroxide (100mL) and the organic layer was separated and dried (Na₂SO₄). The solution was filtered and evaporated under reduced pressure to the point where a solid began to precipitate. At this point hexanes were added causing precipitation of the product which was collected by filtration and dried under high vacuum.

### 2. Specific Experimental Examples Of Syntheses

### Example 1: 4-Hydroxyindan

4-Aminoindan (3.0g) was added to a solution of concentrated sulphuric acid (2.4mL) in water (15mL). More water (15mL) was added and the mixture cooled to 5°C. A solution of sodium nitrite (1.71g) in water (4.5mL) was added portionwise to the mixture while maintaining the temperature below 5°C. After addition was complete the mixture was allowed to warm to room temperature and urea (0.29g) was added. The mixture was stirred for a further 5 minutes before being heated at 45°C for 30 minutes. The mixture was then cooled to room temperature and extracted with ethyl acetate. The combined organic extracts were washed with 2M aqueous sodium hydroxide (2x100mL) and these aqueous extracts were then acidified with hydrochloric acid and extracted with ethyl acetate (3x100mL). The combined organic extracts were then washed with brine and dried (Na₂SO₄) before being concentrated *in vacuo.* The resulting crude product was chromatographed over silica gel. Elution with ethyl acetate/hexanes (1:7) gave 4-hydroxyindan as an orange oil (1.0g).

### Example 2: 4-Indanyl triflate

To a solution of 4-hydroxyindan (1.2g, 8.9mmol) in pyridine (5mL) at 0°C was slowly added trifluoromethanesulphonic anhydride (1.6mL, 9.8mmol). The resulting mixture was stirred at 0°C for 5 minutes before being allowed to warm to room temperature and then stirred for 45 minutes. The mixture was then poured into water and extracted with ethyl acetate (3 x 25mL). The combined extracts were washed sequentially with water, 1M aqueous hydrochloric acid, water and brine, then dried (Na₂SO₄) and concentrated *in vacuo* to give the crude triflate as an orange oil (2.13g, 89%).

### Example 3: Methyl 3-(indan-4-yl)acrylate

A mixture of crude 4-indanyl triflate (2.13g, 8.0mmol), methyl acrylate (1.01mL, 11.2mmol), triethylamine (4.4mL, 32mmol, 4eq) and
dichlorobis(triphenylphosphine)palladium (170mg 0.24mmol) in dimethylformamide (15mL) was heated at 85°C for 71 hours. A small aliquot was removed and worked up for GC/MS analysis which revealed a significant amount of starting material was still present. Additional methyl acrylate (0.7mL), triethylamine (2mL) and the palladium catalyst (170mg) were added and the mixture was heated for a further 24 hours. The mixture was then poured into water, extracted with ethyl acetate, and the organic extracts were washed with water, then brine, dried (Na₂SO₄), and concentrated *in vacuo* to give the crude product as an oil (2.4g). The crude product was chromatographed over silica gel. Elution with ethyl acetate/hexanes (1:19) gave the starting triflate (812mg, 38%) as a colourless oil, followed by the desired methyl 3-(indan-4-yl)acrylate as a brown oil (880mg, 54%).

### Example 4: Methyl 3-benzoylcinnamate

To a mixture of 3-bromobenzophenone (5.0g, 19mmol), palladium acetate (215mg, 0.958mmol), and tri-o-tolylphosphine (290mg, 0.953mmol) was added triethylamine (3.3mL, 45mmol), toluene (4mL), and methyl acrylate (2.2mL, 27mmol). The mixture was heated at 100°C for 18 hours at which time -TLC analysis showed the reaction was still incomplete. Additional portions of palladium acetate (215mg, 0.958mmol), tri-o-tolylphosphine (290mg, 0.953mmol), triethylamine (3.3mL, 45mmol) and methyl acrylate (2.2mL, 27mmol) were added, and the mixture was heated at 110° for a further 18 hours. After cooling to room temperature the mixture was poured into water and extracted with ethyl acetate (3 x 100mL). The combined organic extracts were washed sequentially with water and brine, and then dried (MgSO₄) and concentrated to a brown oil (5.3g). The oil was chromatographed over silica gel. Elution with ethyl acetate/hexanes (1:9) afforded methyl 3-benzoylcinnamate (4.6g, 91%) as a yellow solid.

### Example 5: 3-Benzoylcinnamic acid

Aqueous 5M potassium hydroxide (10mL, 50mmol) was added to a solution of methyl 3-benzoylcinnamate (2.5g, 9.4mmol) in methanol (20mL) and the mixture was stirred at room temperature for 18 hours. The mixture was concentrated and acidified to pH 1 using 1M aqueous hydrochloric acid. The resulting precipitate was collected by filtration and dried under vacuum to give 3-benzoylcinnamic acid (2.2 g, 93%) as a yellow solid.

### Example 6: 5-(1-Methyl-1H-pyrazol-4-yl)-2-naphthoic acid

A mixture of 5-bromo-2-naphthoic acid (2.12g, 8.44mmol), 1-methyl-4-(4,4,5,5-tetramethyl-1,3,2-dioxaborolan-2-yl)-1*H*-pyrazole (1.84g, 8.86mmol), and tetrakis(triphenylphosphine)palladium(0) (502mg, 0.435mmol) in a 250mL round bottomed flask was evacuated and purged with nitrogen (in three cycles). Acetonitrile (40mL) and 2M aqueous sodium carbonate (10mL) were added to the mixture via syringe, and the mixture was heated under reflux under nitrogen for 22 hours. The reaction mixture was allowed to cool before the addition of 1M aqueous hydrochloric acid (30mL) and it was then extracted with ethyl acetate (3 x 50mL). The combined organic layers were dried (MgSO₄), filtered, and concentrated *in vacuo* to provide a crude product (2.98g after air drying). This crude material was dissolved in hot ethanol (150mL) and filtered while hot to remove a yellow impurity (120mg). The filtrate was concentrated *in vacuo* and the residue was recrystallised from dichloromethane (30mL) to provide 5-(1-methyl-1*H*-pyrazol-4-yl)-2-naphthoic acid as a white solid (724mg, 34%). A second crop of 5-(1-methyl-1*H*-pyrazol-4-yl)-2-naphthoic acid (527mg, 25%) was obtained from the concentrated mother liquors by recrystallisation from dichloromethane (20mL).

### Example 7: 5-(1-Methyl-1H-pyrazol-4-yl)-2-naphthoylguanidine

Oxalyl chloride (1.1mL, 13mmol) was added to a solution of 5-(1-methyl-1*H-*pyrazol-4-yl)-2-naphthoic acid (1.19g, 4.71mmol) in anhydrous dichloromethane (200mL (which was added in portions during the reaction to effect dissolution)) containing dimethylformamide (2 drops) under nitrogen and the mixture was stirred at room temperature for 4.25 hours. The reaction mixture was then heated for 1 hour at 40°C, before being concentrated under reduced pressure. The resulting crude acid chloride was suspended in anhydrous tetrahydrofuran (50mL) and this mixture was added dropwise to a solution of guanidine hydrochloride (2.09g, 21.9mmol) in 2M aqueous sodium hydroxide (15mL, 30mmol) and the reaction mixture was then stirred for 30 minutes. The organic phase was separated, and the aqueous phase was extracted with chloroform (3 x 30mL) followed by ethyl acetate (3 x 30mL). The combined organic extracts were washed sequentially with 1M aqueous sodium hydroxide (60mL) and water (40mL), then dried (Na₂SO₄) and concentrated *in vacuo* to give a glassy solid (1.45g after drying under high vacuum). This solid was dissolved in dichloromethane which was then allowed to evaporate slowly to give 5-(1-methyl-1*H*-pyrazol-4-yl)-2-naphthoylguanidine as a yellow solid (1.15g, 83%).

### Example 8: Ethyl 2,3-methylenedioxycinnamate

Triethyl phosphonoacetate (4.05mL, 20.2mmol) was added dropwise to a stirred suspension of sodium hydride (0.80g, 20mmol) in anhydrous tetrahydrofuran (20 mL) at 0°C under nitrogen. The mixture was stirred at 0°C for 20 minutes. A solution of 2,3-methylenedioxybenzaldehyde (2.50g, 16.7mmol) in tetrahydrofuran (10mL) was added dropwise at 0°C. The mixture was stirred for 2 hours during which time it was allowed to warm to room temperature. The mixture was poured into water (250mL), and extracted with ethyl acetate (3 x 250mL). The combined organic extracts were then washed with brine, dried (MgSO₄) and concentrated *in vacuo.* The crude product was chromatographed over silica gel. Elution with ethyl acetate/hexanes (1:10) gave ethyl 2,3-methylenedioxycinnamate as a colourless solid (3.50g, 92%).

### Example 9: 2,3-Methylenedioxycinnamic acid

A solution of ethyl 2,3-methylenedioxycinnamate (3.40g) in methanol (25mL) and water (5mL) was treated with a solution of potassium hydroxide (4.3g) in water (25mL). The mixture was stirred overnight at room temperature before being concentrated *in vacuo* to half its original volume. The concentrate was then acidified with concentrated HCl to give 2,3-methylenedioxycinnamic acid as a colourless solid (2.81g, 95%) which was collected by filtration and dried overnight under a vacuum.

### Example 10: 2,3-Methylenedioxycinnamoylguanidine

Oxalyl chloride (0.68mL, 7.8mmol) was added to a suspension of 2,3-methylenedioxycinnamic acid (500mg, 2.6mmol) in dichloromethane (5mL) containing a drop of dimethylformamide. The mixture was stirred for 2.5 hours and the resulting solution was evaporated to dryness under reduced pressure. The residue was dissolved in dry tetrahydrofuran (5mL) and added to a solution of guanidine hydrochloride (1.24g, 13mmol) in 2M aqueous sodium hydroxide (8mL). The reaction was stirred at room temperature for 1hour and chloroform was then added. The resulting precipitate of crude product (100mg) was collected by filtration. The filtrate was extracted with chloroform (3 x 30mL) and ethyl acetate (20mL). The combined organic extracts were washed with 2M aqueous sodium hydroxide (20mL), water (20mL), dried (Na₂SO₄) and concentrated under reduced pressure to give a further quantity of crude product (400mg). The two crops of crude product were combined, suspended in chloroform (10mL) and stirred vigorously for 20 minutes. The resulting 2,3-methylenedioxycinnamoylguanidine (420mg) was collected by filtration and dried under vacuum.

### Example 11: Anti-viral activity of compounds using the bacterial bioassay method

The bacterial bioassay method used in the present example to test the anti-viral activity of the compounds against different viral targets was described in detail in PCT/2004/000866. This assay is used in conjunction with the GBV-B and BVDV assays described below, to ensure that all active compounds are identified, some of which are active in one or the other of the assays, while some compounds may be active in both assays.

Briefly, the bacterial bio-assay for screening potential anti- HCV compounds is based on the HCV p7 ion channel protein. p7 is a small membrane protein encoded by HCV, which has a functional activity supporting viral growth and/or replication.

The p7-encoding synthetic cDNA fragment cDp7.coli, in which codons were optimised for expression of the p7 protein in E.coli, was cloned into the expression plasmid pPL451, creating the vector pPLp7, in which p7 expression is temperature inducible, as described in detail in PCT/2004/000866. Inhibition of the growth of E.coli cells expressing p7 at 37°C was observed as an indicator of p7 ion channel function dissipating the normal Na+ gradient maintained by the bacterial cells. Halos of growth around a particular compound application site indicate that the compound has inhibited expression of the p7 ion channel activity that prevents growth in the absence of the compound.

The cumulative results of the bacterial bioassay tests obtained over a period of time and averaged, are summarised in Table 1 below.

**Table 1: Mean Bacterial Bioassay Assay Scores For Compounds Of The Invention**

| **Compound Name** | **BIT#** | **Average Bacterial Assay Score HCV p7** |
|---|---|---|
| (3-benzoyl)cinnamoylguanidine | 216 | 1.3 |
| 2,3-methylenedioxycinnamoyl guanidine | 217 | 1.0 |
| 5-methyl-2-napthoylguanidine | 218 | 1.7 |
| 3(indan-4-yl)-propenoylguanidine | 222 | 2.0 |
| 5-bromo-6-methoxy-2-napthoylguanidine | 223 | 0.5 |
| 5-thiophen-3-yl-2-naphthoylguanidine | 224 | 1.10 |
| 5-(1-methylpyrazol-4-yl)2-naphthoylguanidine | 225 | 1.20 |
| 3,4-dichlorocinnamoyl guanidine | 300 | 1.12 |
| (1-methoxy-2-napthoyl)guanidine | 301 | 0.25 |
| (3-methoxy-2-napthoyl)guanidine | 302 | 0.76 |
| (5-bromo-2-napthoyl)guanidine | 303 | 0.62 |
| (1,4-dimethoxy--2-napthoyl)guanidine | 304 | 0.60 |
| (6-(3-thienyl)-2-napthoyl)guanidine | 305 | 0.08 |
| (6-methyl-2-napthoyl)guanidine | 306 | 0.07 |
| (5-phenyl-2-napthoyl)guanidine | 307 | 0.46 |
| (5-(thien-2-yl)-2-napthoyl)guanidine | 308 | 0.55 |
| (5-(1-isobutyl-1H-pyrazol-4-yl)-2-napthoyl)guanidine | 310 | 0.36 |
| (5-(3-furyl)-2-napthoyl)guanidine | 311 | 0.81 |
| (5-cyclopropyl-2-napthoyl)guanidine | 312 | 1.00 |
| (5-chloro-2-napthoyl)guanidine | 313 | 1.30 |
| (6-(1-methylpryazol-4-yl)-2-napthoyl)guanidinium acetate | 314 | 4.03 |
| (5-(2,6-dimethoxypryridin-3-yl)-2-napthoyl)guanidine | 315 | 0.20 |
| (5-(2-chlorophenyl)-2-napthoyl)guanidine | 316 | 0.37 |
| (5-(4-(acetylamino)phenyl)-2-napthoyl)guanidine | 317 | 0.06 |
| (5-(3-(acetylamino)phenyl)-2-napthoyl)guanidine | 318 | 0.73 |
| (5-(4-((methylsulphonyl)amino)phenyl)-2-napthoyl)guanidine | 319 | 0.10 |
| | | |
| ASSAY POSITIVE CONTROL | | |
| (3-Bromocinnamoyl)guanidine | BIT06 7 | 2.00 |
| 5-bromo-2-fluorocinnamoylguanidine | BIT12 4 | 2.70 |

The positive controls were used in this assay to ensure that the assay was working rather than for comparison of relative activities of the compounds. A result above zero indicates that the compound has potential anti-viral activity.

### Example 12 - Testing p7 inhibitors against Hepatitis C Virus.

Testing of the antiviral efficacy of new potential HCV drugs is made difficult by the lack of a generally accessible cell culture model system for HCV. Proposed p7 inhibitors were tested using surrogate flavivirus systems, in particular GBV-B and BVDV (Bovine Viral Diarrhoea Virus) systems.

GBV-B is the most closely related flavivirus to HCV, sharing 27-33% nucleotide sequence identity and 28% amino acid similarity over the complete polypeptide sequence. This virus represents an excellent surrogate system for HCV because it infects small New World primates and replicates efficiently *in vitro* in primary marmoset hepatocyte (PMH) cultures. The GBV-B homologue of HCV p7 is called p13. It is shown herein that a synthetic peptide corresponding to the two C-terminal transmembrane helices of p13 (which share the greatest homology to p7) forms a cation selective ion channel that, like the p7 channel, is blocked by amantadine (Premkumar *et al*., 2006). On the other hand, unlike p7, HMA does not inhibit the p13 channels. These observations confirm that the two homologous channels share similar, but not identical, structural features.

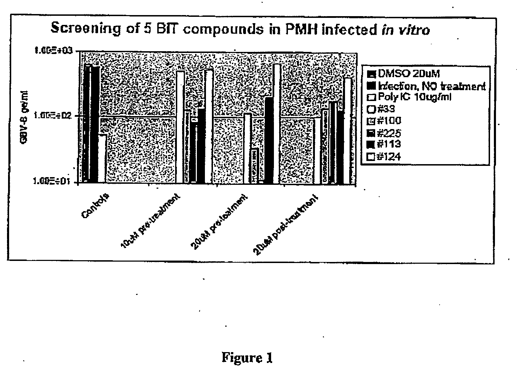

Selected BIT compounds - identified by bacterial assay screening for inhibitors of HCV p7 - were tested for ability to inhibit GBV-B replication in primary marmoset hepatocytes (see Figure 1). The hepatocytes were inoculated 3-days after plating with 10x TCID₅₀ of GBV-B positive marmoset serum. HCV was adsorbed for two hours, and then the cells were washed three times and cultured for two days in fresh serum-free medium (SFM) supplemented with hormones and growth factors. Virus released to the culture supernatant was measured as viral RNA copy number, as determined by real-time RT-PCR. Compounds - dissolved in DMSO - were added to the medium either 30 min prior to virus inoculation ("pre-treatment"), or immediately after the inoculation and washing steps ("post-treatment"). No treatment, negative (DMSO only) and positive (10µg/ml Poly I:C) controls were included in the experiments. Cytotoxicity of the compounds toward the hepatocytes was tested via a standard MTT assay.

The most striking result was in the cells pre-treated with 20µM BIT225, in which no virus was detected in the culture supernatant. 20µM BIT100 reduced virus replication by more than 1.0 log and inhibited virus more strongly than the poly I:C positive control. The efficacy of both BIT225 and BIT100 were reduced somewhat when the compounds were added post-inoculation, suggesting that the compounds may act at a very early stage of the virus life-cycle. None of the compounds in Figure 1 showed cytotoxicity to the hepatocytes at 20µM

BVDV belongs to the Pestivirus genus of Flaviviridae and is widely used as a surrogate model system for identification of potential HCV antiviral agents due to the many similarities of their genome structure, gene products and replication cycles. In addition, unlike GBV-B, which can only be cultured in primary hepatocytes, BVDV is readily grown in tissue culture and commonly used strains are cytopathic, making for easy testing of antiviral drugs. The HCV p7 homologue of BVDV has been shown to form an ion channel and to be essential for generation of infectious virus particles (Harada *et al*., 2000 and Griffin *et al*., 2005).

The antiviral evaluation of selected BIT compounds against BVDV was outsourced to Southern Research Institute (SRI), Fredrick MD USA. A simple cytoprotection format is used in which the antiviral efficacy of the compounds is assessed by their ability to reduce the cytopathic effect of BVDV infection in Madin-Darby bovine kidney cells (Buckwold *et al*., 2003)

A virus-induced cytopathogenic effects (CPE)-inhibition assay procedure was employed to evaluate compounds for antiviral activity against bovine viral diarrhea virus (BVDV) strain NADL, in Madin-Darby bovine kidney (MDBK) cells passaged in T-75 flasks (1, 2). Antiviral assays were designed to test six half-log concentrations of each compound in triplicate against the challenge virus. Cell controls (CC) containing medium alone, virus-infected cell controls (VC) containing medium and virus, drug cytotoxicity controls containing medium and each drug concentration, reagent controls containing culture medium only (no cells), and drug colorimetric controls containing drug and medium (no cells) are run simultaneously with the test samples. Human interferon-α 2b was used as a positive control compound. On the day preceding the assay, the cells were 4 trypsinized, pelleted, counted and resuspended at 1x10 / well in tissue culture medium in 96-well flat bottom tissue culture plates in a volume of 100 µl per well. One day following platingof cells, the wells were washed and the medium was replaced with complete medium (2% serum) containing various concentrations of test compound diluted in medium in a half-log series. A pre-titered aliquot of virus was removed from the freezer (-80°C) just before each experiment. The virus was diluted into tissue culture medium such that the amount of virus added to each well would give complete cell killing at 6-7 days post-infection. The plates were incubated at 37°C in a humidified atmosphere containing 5% CO₂ until maximum CPE is observed in the untreated virus control cultures (∼day 7). Inhibition of CPE by the compound was determined using Cell Titer 96 (Promega). A colorimetric method for determining the number of viable cells was used. A computer program was utilized to calculate the percent of CPE reduction of the virus-infected wells and the percentage cell viability of uninfected drug control wells. The minimum inhibitory drug concentration which reduces the CPE by 50% (IC₅₀) and the minimum toxic drug concentration which causes the reduction of viable cells by 50% (TC₅₀) were calculated using a regression analysis program with semi log curve fitting. A therapeutic (selectivity) index (TI₅₀) for each active compound was determined by dividing the TC₅₀ by the IC₅₀.

Drug cytotoxicity was measured separately in uninfected cells. Eleven BIT compounds were tested in the first experiment in which the compounds were added to the cells just prior to infection and were maintained throughout the entire experiment. Two of them, BIT225 and BIT314 returned sub-micromolar IC₅₀ values (see Table 2 below).

**Table 2 - Antiviral Efficacy vs. BVDV in MDBK Cells**

| **Compound** | **IC₅₄** | **TC₅₉** | **Al** |
|---|---|---|---|
| BIT-225 | 0.53 µM | 11.6 µM | 21.7 |
| BIT-300 | N/A | 3.69 µM | N/A |
| BIT-124 | N/A | 5.21 µM | N/A |
| BIT-33 | 3.64 µM | 25.2 µM | 6.91 |
| BIT-143 | 4.66 µM | 17.0 µM | 3.65 |
| BIT-93 | 16.7 µM | >30.0 µM | >1.80 |
| BIT-123 | N/A | 9.92 µM | N/A |
| BIT-137 | N/A | 16.5 µM | N/A |
| BIT-110 | N/A | 16.8 µM | N/A |
| BIT-314 | 0.21 µM | 11.6 µM | 54.2 |
| BIT-223 | 4.38 µM | >30.0 µM | >6.85 |
| IFN-α | 20.6 IU/mL | >500 IU/mL | >24.3 |

| | | | |
|---|---|---|---|
| N/A = not achieved | | | |

A subsequent repeat assay with BIT225 returned a similar IC₅₀ value of 0.33µM. Additional compounds of the invention were also tested, as shown in Table 2a below.

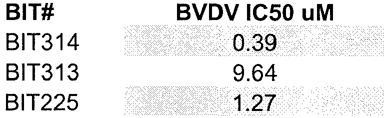

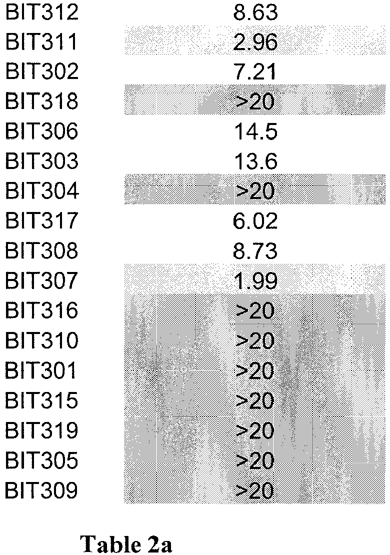

### Example 13 - Inhibition of HCV using a combination of BIT225 with IFN or Ribavirin.

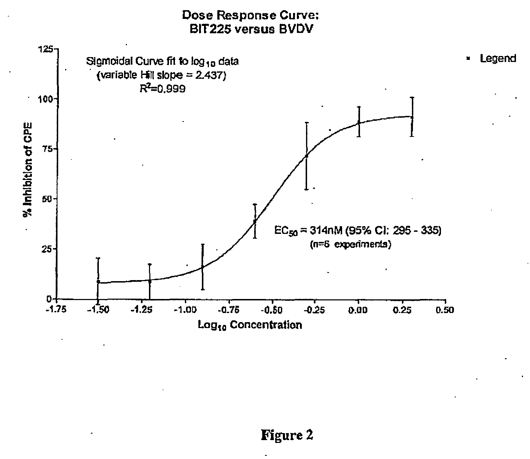

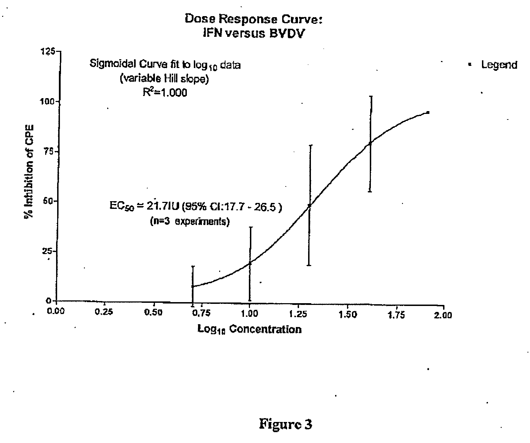

Combinations of BIT225/IFN and BIT225/Ribavirin were tested against the virus. Figures 2, 3 and 4 show that each drug individually yielded the following EC₅₀ values: BIT225, 314nM; rIFNα-2b, 21.7 IU/ml; but for Ribavirin on its own, only very little antiviral activity was detected in the range up to 20µg/ml.

The effects of drug combinations were calculated on the activity of each compound when tested alone. The expected additive antiviral protection was subtracted from the experimentally determined antiviral activity at each combination concentration resulting in a positive value (synergy), a negative value (antagonism), or zero (additivity). The synergy volume (in units of concentration times concentration times percent, for example, µM2%, nM2%, nMµM%, and the like) was calculated at the 95% confidence interval. For these studies, synergy was defined as drug combinations yielding synergy volumes greater than 50. Slightly synergistic activity and highly synergistic activity have been operationally defined as yielding synergy volumes of 50-100 and >100, respectively. Additive drug interactions have synergy volumes in the range of -50 to 50, while synergy volumes between -50 and -100 are considered slightly antagonistic and those < -100 are highly antagonistic.

Table 3 summarizes the results of the combination studies: BIT225 and IFN had an average synergy volume of 87 IU/mlµM% indicating slight synergy, although note that the value is close to the "highly synergistic" cut-off and in one of the three experiments the interaction was found to be highly synergistic. Interestingly, the BIT225/Ribavirin combination was slightly antagonistic. In these experiments, where Ribavirin on its own had no antiviral activity, the result indicates that Ribavirin was antagonizing the strong antiviral activity of BIT225.

Although there was an attempt herein to "grade" the degree of synergy between different combinations of antiviral compounds, it will be understood that the term "synergy" is also commonly used in its absolute sense and hence any level of synergy is considered relevant and significant with respect to the combinations of the present invention.

**Table 3 - BVDV Combination Assay**

| ***Compounds*** | | ***Combination Scheme*** | | |
|---|---|---|---|---|
| BIT225 & rIFNα-2b | | 8 2-fold dilutions of BIT225; high-test concentration at 4µM | | |
| BIT225 & Ribavirin | | 8 2-fold dilutions of BIT225; high-test concentration at 4µM | | |
| | | 5 2-fold dilutions of Ribavirin; high-test concentration at 20µg/mL | | |
| | | | | |

| | | **Antiviral Efficacy** | **Cytotoxicicity** | |
|---|---|---|---|---|
| **Assay** | **Synergy/Antago nism** | **Interpretation** | **Synergy/Antagonism Volume (1U/mLnM%)** | **Interpretati on** |
| BIT225 & rIFNα-2b; 1st | 72/ -2 IU/mLµM% | Slightly synergistic | 3 / -2 IU/mLµM% | Additive |
| BIT225 & rIFNα-2b; 2nd | 106/0 IU/mLµM% | Highly synergistic | 0 / -10 IU/mLµM% | Additive |
| BIT225 & rIFNα-2b; 3rd | 84 / 0 IU/mLµM% | Slightly synergistic | 0 / -9 IU/mLµM% | Additive |
| **BIT225 & rIFNα-2b; avg** | **87 / -1 IU/mLµM%** | **Slightly synergistic** | **1 / -7 IU/mLµM%** | ***Additive*** |
| BIT225 & Ribavirin; 1st | 4/-62 µg/mLµM% | Slightly antagonistic | 4 / -7 µg/mLµM% | Additive |
| BIT225 & Ribavirin; 2nd | 0/-89 | Slightly antagonistic | 0 / -6 µg/mLµM% | Additive |
| BIT225 & Ribavirin; 3rd | 0/-65 | Slightly antagonistic | 3 / 0 µg/mLµM% | Additive |
| ***BIT225 &*** | **1 / -72 µg/mLµM%** | **Slightly antagonistic** | **2 / -4 µg/mLµM%** | ***Additive*** |
| ***Ribavirin; avg*** | | | | |

### Example 14 - Inhibition of HCV using a combination of BIT225, IFN and Ribavirin

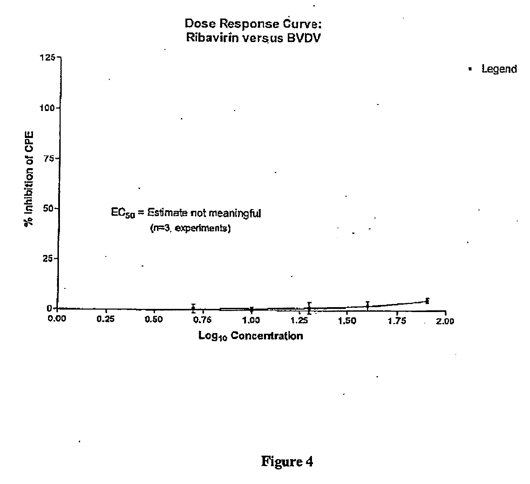

The results of the above combination studies revealed synergism between the antiviral activities of BIT225 and IFNα. It is also well known from the literature that although Ribavirin has very little activity against BVDV on its own (see Figure 4), the compound enhances the antiviral activity of IFNα. Interestingly, although a slight antagonism was reported between ribavirin and BIT225 (see Table 3), this was predominantly seen at the higher concentrations of both drugs tested.

The effect of a combination of BIT225, IFNα and Ribavirin was tested. Two fixed, sub EC₅₀ concentrations of IFNα (5 and 10 IU/ml) were chosen and tested against varying concentrations of BIT225 and Ribavirin: 8 two-fold dilutions of BIT225 from a high-test concentration of 4µM and 5 two-fold dilutions of Ribavirin from a high-test concentration of 20µg/ml were tested. The results, presented in Table 4, show highly synergistic antiviral activities between BIT225 and Ribavirin at both fixed concentrations of IFNα tested.

**Table 4**

| **Compounds** | | **Combination Scheme** | | | |
|---|---|---|---|---|---|
| Fixed concentration of rIFNα-2b (5 IU/mL) combining with varying amounts of BIT-225 & Ribavirin | | Fixed con Fixed concentration of rIFNα-2b (5 IU/mL); | | | |
| | | 5 2-fold dilutions of Ribavirin; high test concentration at 20µg/mL; | | | |
| | | 8 2-fold dilutions of BIT225; high-test concentration at 4µM | | | |
| Fixed concentration of rIFNα-2b (10 IU/mL) combining with varying amounts of BIT-225 & Ribavirin | | Fixed con Fixed concentration of rIFNα-2b (10 IU/mL); | | | |
| | | 5 2-fold dilutions of Ribavirin; high test concentration at 20µg/mL; | | | |
| | | 8 2-fold dilutions of BIT225; high-test concentration at 4µM | | | |
| | | | | | |

| | | **Antiviral Efficacy** | | **Cytotoxicity** | |
|---|---|---|---|---|---|
| **Assay** | **Synergy/Antagonism Volume** | | **Interpretation** | **Synergy/Antagonism Volume** | **Interpretation** |
| Fixed concentration of rIFNα-2b (5 IU/mL) combining with varying amounts of BIT-225 & Ribavirin | | 138 / 0 µg/mLµM% | Highly synergistic | 0 / -59 µg/mLµM% | Slightly antagonistic |
| Fixed concentration of rIFNα-2b (10 IU/mL) combining with varying amounts of BIT-225 & Ribavirin | | 127 / 0 µg/mLµM% | Highly synergistic | 0 / -67 µg/mLµM% | Slightly antagonistic |

Further analysis of the data reveal 70% inhibition of viral CPE for the combination of 5 IU/ml IFNα plus the lowest concentration of BIT225 tested (31nM), plus the lowest concentration of Ribavirin tested (1.25 µg/ml). The same low concentrations of BIT225 and Ribavirin, in the presence of 10 IU IFNα yielded 90% virus inhibition. For comparison, from the earlier studies 5 IU/ml IFNα alone gives ∼8% inhibition; 31 nM BIT225 alone gives ∼5% inhibition; and 1.25µg/ml Ribavirin alone shows no antiviral activity. Clearly the triple combination is highly efficacious against BVDV.

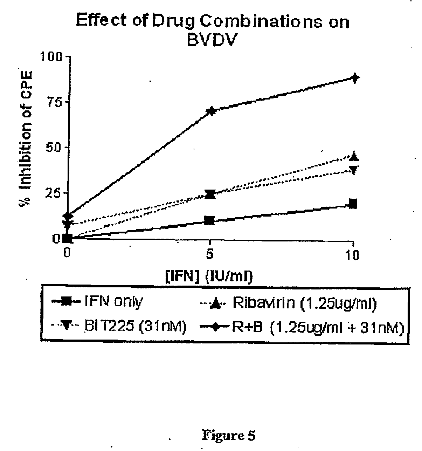

Figure 5 shows the levels of virus inhibition seen with 31nM BIT225 and/or 1.25µg Ribavirin in the presence of absence of IFNα.

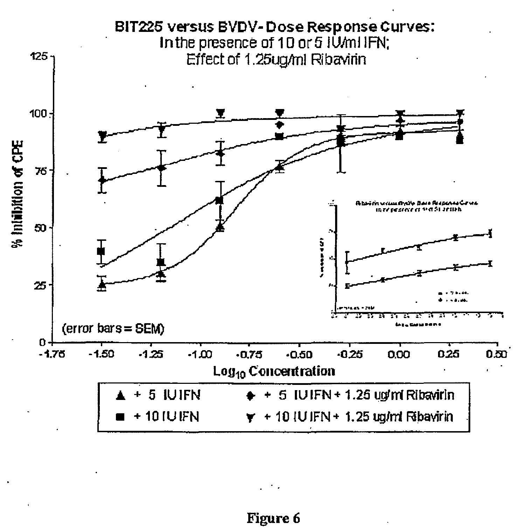

Figure 6 shows the full-range dose response curves for BIT225 in the presence of 5 and 10 IU/m IFNα and shows the enhanced antiviral effect by addition of 1.25 µg/ml. The inset shows the full range dose response curves for Ribavirin in the presence of 5 and 10 IU/m IFNα.

The EC₅₀ values for BIT225 in the presence of 5 or 10 IU/ml IFNα were determined as 92 (95% CI: 22 - 385) nM and 71 (95% CI: 41 - 1240) nM, respectively, by standard sigmoidal curve fitting performed with Prism software with Hill slope constrained. Similar curve fitting allowing a variable Hill slope yields equivalent EC₅₀ values or 149 and 125 nM, in good agreement with the values determined in the previous experiment.

It was not possible to determine EC₅₀ values for the data from experiments in which Ribavirin was added because all drug combinations tested yielded >70% inhibition of viral CPE.

### Summary

The combination studies with compound BIT225 show that:
- BIT225 alone has good antiviral activity with an EC₅₀ value of 314nM (95% CI: 295-333).
- BIT225 shows synergism in combination with IFNα; the EC₅₀ value of BIT225 in the presence of 5 IU/ml IFNα is lowered to ∼92nM (95% CI: 22 - 385).
- The triple combination of BIT225, IFNα and Ribavirin is strongly synergistic, yielding 70% inhibition of virus CPE with as low as 31nM, BIT225, 5 IU/m IFNα and 1.25µg/ml Ribavirin.
- Complete virus inhibition can be achieved with various combinations of the three compounds: For example; 5 IU/ml IFNα + 500nM BIT225 + 2.5µg/ml Ribavirin, or; 10 IU/ml IFNα + 31nM BIT225 + 2.5µg/ml Ribavirin.

### Example 15 - Inhibition of HCV using a reference combination of BIT225 with Nucleoside Analogs 2'-C-methyladenosine or 2'-C-methylcytidine.

The nucleoside analogues described herein may be synthesised using protocols described in Hecker SJ et al (2007) J. Med Chem. 50(16), 3891-6 (for 2'-C-methyladenosine) and Antiviral Research (2007) 73(3), 161-8 (for 2'-C-methylcytidine). Nucleoside analogues can also be obtained from commercial sources such as NANJING BAIFULI TECHNOLOGY CO., LTD.(NAN JING BAI FU LI KE JI YOU XIAN ZE REN GONG SI), RM 701 , BLDG 15, High-Tech Zone, Nanjing, 210061, P.R.CHINA

**Table 5 - BVDV Combination Assay**

| **Compounds** | | **Combination Scheme** | | |
|---|---|---|---|---|
| BIT225 & 2'-C-methyladenosine | | 8 2-fold dilutions of BIT225; high-test concentration at 4µM | | |
| | | 5 2-fold dilutions of 2'-C-methyladenosine; high-test concentration at 10 µM | | |
| BIT225 & 2'-C-methylcytidine | | 8 2-fold dilutions of BIT225; high-test concentration at 4µM | | |
| | | 5 2-fold dilutions of 2'-C-methylcytidine; high-test concentration at 10 µM | | |
| BIT314 & rIFNα-2b | | 8 2-fold dilutions of BIT314; high-test concentration at 4µM | | |
| | | 5 2-fold dilutions of rIFNα-2b; high-test concentration at 80 IU/mL | | |
| Fixed concentration of rIFNα-2b (5 IU/mL) combining with varying amounts of BIT-314 & Ribavirin | | Fixed con Fixed concentration of rIFNα-2b (5 IU/mL); | | |
| | | 8 2-fold dilutions of BIT-314; high test concentration at 4µM; | | |
| | | 5 2-fold dilutions of Ribavirin; high-test concentration at 20µg/mL | | |
| | | | | |
| | | | | |

| | **Antiviral Efficacy** | | **Cytotoxicity** | |
|---|---|---|---|---|
| **Assay** | **Synergy/Antagonism Volume** | **Interpretation** | **Synergy/Antagonism Volume** | **Interpretation** |
| BIT225 & 2'-C-methyladenosine | 106.62/-3.41 µM²% | Highly synergistic | 1.91/ -53.29 µM²% | Slightly antagonistic |
| BIT225 & 2'-C-methylcytidine | 71.23/0 µM²% | Slightly synergistic | 0/-18.31µM²% | additive |
| BIT314 & rIFNα-2b | 311.42 /-2.11 µMIU/mL% | Highly synergistic | 0/-1.84µMIU/mL% | Additive |
| Fixed concentration of rIFNα-2b (5 IU/mL) combining with varying amounts of BIT-314 & Ribavirin | 361.68/-12.19 µMµg/mL% | Highly synergistic | 22.65/-0.34 µMug/mL% | Additive |

Table 5 above summarises the results of combination studies with compound BIT225 and nucleoside analogs, and compound BIT314 combinations with IFN and/or ribavirin.

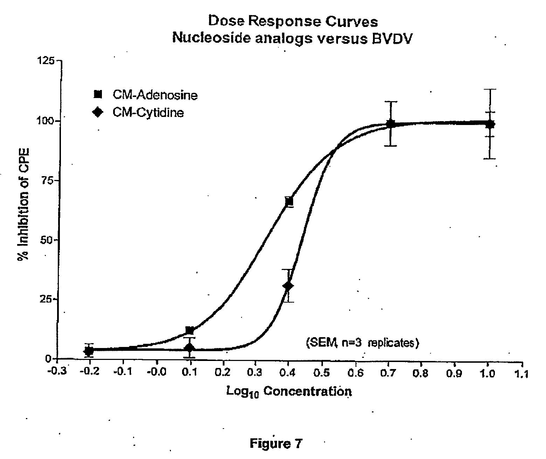

As shown herein, combinations of BIT225/2'-C-methyladenosine and BIT225/2'-C-methylcytidine were tested against the virus. Figure 7 shows that each nucleoside analog was active individually with the following EC₅₀ values: 2'-C-methyladenosine EC₅₀ = 2.16 µM (95% CI: 1.54 to 3.03 µM); 2'-C-methylcytidine EC₅₀ = 2.75 µM (95% CI: 0.86 to 8.7 µM).

As before, the effects of drug combinations were calculated on the activity of each compound when tested alone. The expected additive antiviral protection was subtracted from the experimentally determined antiviral activity at each combination concentration resulting in a positive value (synergy), a negative value (antagonism), or zero (additivity). The synergy volume (in units of concentration times concentration times percent, for example, µM2%, nM2%, nMµM%, and the like) was calculated at the 95% confidence interval. For these studies, synergy was defined as drug combinations yielding synergy volumes greater than 50. Slightly synergistic activity and highly synergistic activity have been operationally defined as yielding synergy volumes of 50-100 and >100, respectively. Additive drug interactions have synergy volumes in the range of -50 to 50, while synergy volumes between -50 and -100 are considered slightly antagonistic and those < -100 are highly antagonistic.

Table 5 summarizes the results of the combination studies: BIT225 and 2'-C-methyladenosine had an average synergy volume of 106 µM²% indicating "high" synergy. BIT225 and 2'-C-methylcytidine had an average synergy volume of 71 µM²% indicating "slight" synergy.

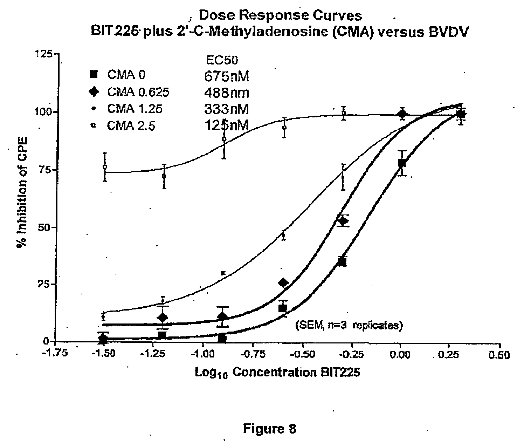

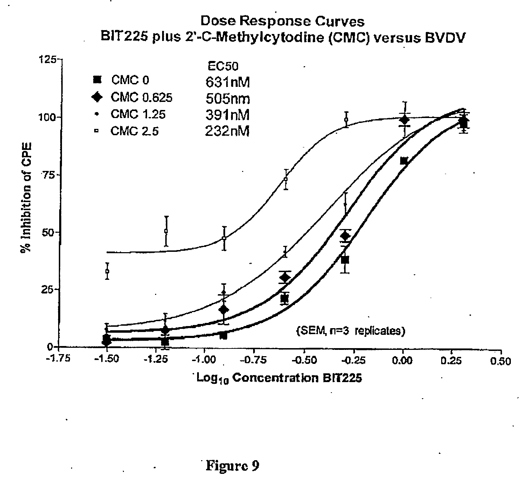

Figures 8 and 9 show the changes to dose response curves for BIT225 in the presence of various concentrations of 2'-C-methyladenosine or 2'-C-methylcytidine, respectively.

### Example 16 - Inhibition of HCV using a combination of BIT314 with IFN.

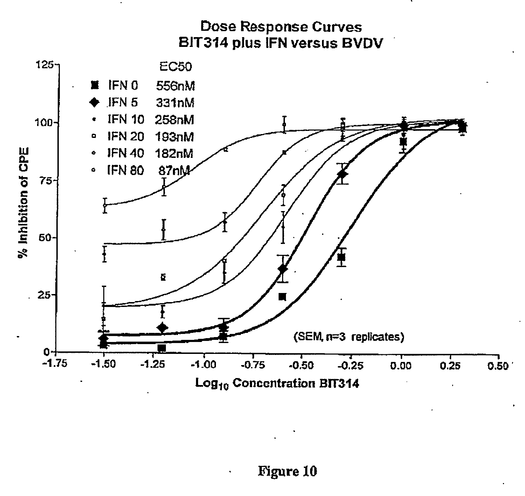

Combinations of BIT314/IFN were tested against the virus. Two previous experiments with BIT314 tested individually against BVDV yielded EC₅₀ values of, 210nM and 390nM (average = 300nM). Similarly, we have previously determined an EC₅₀ value of 21.7 IU/ml for rIFNα-2b. Figure 10 includes the dose response curve for BIT314, as determined in a third experiment, which was part of these combination studies. In that experiment the EC₅₀ for BIT314 was 540 nM.

As previously, the effects of drug combinations were calculated on the activity of each compound when tested alone as described in example 15. Table 5 summarizes the results of the combination studies: BIT314 and IFN had an average synergy volume of 311 µMIU/ml% indicating "high" synergy.

### Example 17 - Inhibition of HCV using triple combinations of BIT314, IFN and Ribavirin

The results of the above combination studies revealed strong synergism between the antiviral activities of BIT314 and IFNα. It is also well known from the literature that although Ribavirin has very little activity against BVDV on its own (see Figure 4), the compound enhances the antiviral activity of IFNα.

The effect of a combination of BIT314, IFNα and Ribavirin was tested. A single fixed - sub EC₅₀ - concentration of IFNα (5 IU/ml) was chosen and tested against varying concentrations of BIT314 and Ribavirin: 8 two-fold dilutions of BIT314 from a high-test concentration of 4µM and 5 two-fold dilutions of Ribavirin from a high-test concentration of 20µg/ml were tested. The results, summarised in Table X, show highly synergistic antiviral activities between BIT314 and Ribavirin in the presence of IFNα: synery/antagonism volume of 361 µMµg/ml%. 4

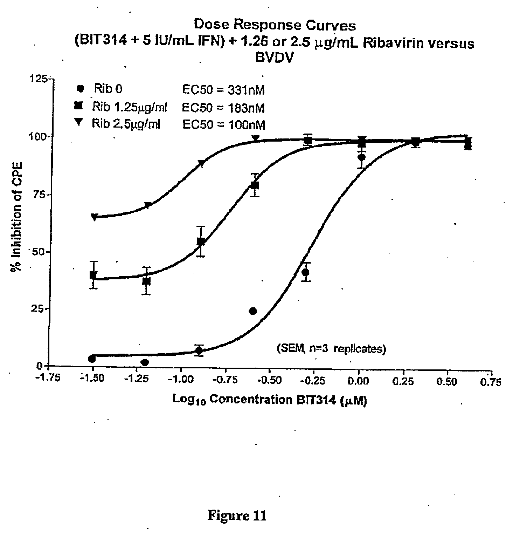

Figure 10 shows full-range dose response curves for BIT314 in the presence of and various concentrations of rIFNα-2b and Figure 11 illustrates the enhanced antiviral effect by addition of 5 IU/m IFNα + 1.25 µg/ml ribavirin and 5 IU/m IFNα + 2.5 µg/ml ribavirin. The EC₅₀ value for BIT314 alone, in this experiment, was 540 nM (95% CI: 389 to 739 nM) and; in the presence of 5 IU/ml IFNα plus 1.25 µg/ml ribavirin was 183 nM (95% CI: 148 to 226 nM), as determined by standard sigmoidal curve fitting performed with Prism software.

### Summary

The combination studies with compound BIT314show that:
- BIT314 alone has good antiviral activity with an average EC₅₀ value of 380nM (SEM 95.4, n=3)).
- BIT314 shows synergism in combination with IFNα; the EC₅₀ value of BIT314 in the presence of 40 IU/ml IFNα is lowered to approximately 60nM.
- The triple combination of BIT314, IFNα and Ribavirin is strongly synergistic, yielding 70% inhibition of virus CPE with as low as 62nM BIT314, 5 IU/m IFNα and 2.5µg/ml Ribavirin.
- Complete virus inhibition can be achieved with various combinations of the three compounds: For example; 5 IU/ml IFNα + 250nM BIT314 + 2.5µg/ml Ribavirin, or; 5 IU/ml IFNα + 500nM BIT314 + 1.25µg/ml Ribavirin.

### References

VanCott TC, Mascola JR, Loomis-Price LD, Sinangil F, Zitomersky N, McNeil J, Robb ML, Birx DL, Barnett S. (1999) J. Virol. 73(6):4640-50
Pauwels R, Balzarini J, Baba M, Snoeck R, Schols D, Herdewijn P, Desmyter J and De Clercq E. (1988) J. Virolog. Methods. 20:309-321
D'Cruz OJ, Shih M-J, Yiv SH, Chen C-L, Uckun FM. (1999) Mol. Hum. Reprod. 5(5):421-432
Joo, Hong-Gu. (2003) J. Vet. Sci. 4(3):229-234
Ewart, G.D., T. Sutherland, P.W. Gage, and G.B. Cox, The Vpu protein of human immunodeficiency virus type 1 forms cation-selective ion channels. J Virol, 1996. 70(10): p. 7108-15.
Ewart, G.D., K. Mills, G.B. Cox, and P.W. Gage, Amiloride derivatives block ion channel activity and enhancement of virus-like particle budding caused by HIV-1 protein Vpu. Eur Biophys J, 2002. 31(1): p. 26-35.
Ewart, G.D., N. Nasr, H. Naif, G.B. Cox, A.L. Cunningham, and P.W. Gage, Potential new anti-human immunodeficiency virus type 1 compounds depress virus replication in cultured human macrophages. Antimicrob Agents Chemother, 2004. 48(6): p. 2325-30.
Gage, P., G. Ewart, J. Melton, and A. Premkumar, Virus Ion Channels Formed by Vpu of HIV-1, the 6K Protein of Alphaviruses and NB of Influenza B Virus., in Viral Membrane Proteins: Structure, Function and Drug Design, W. Fischer, Editor. 2005, Kluwer Academic / Plenum Publishers: New York. p. Chapter 15.
Melton, J.V., G.D. Ewart, R.C. Weir, P.G. Board, E. Lee, and P.W. Gage, Alphavirus 6K proteins form ion channels. J Biol Chem, 2002. 277(49): p. 46923-31.
Premkumar, A., X. Dong, G. Haqshenas, P.W. Gage, and E.J. Gowans, Amantadine inhibits the function of an ion channel encoded by GB virus B, but fails to inhibit virus replication. Antivir Ther, 2006. 11(3): p. 289-95.
Premkumar, A., C.R. Horan, and P.W. Gage, Dengue virus Mprotein C-terminal peptide (DVM-C) forms ion channels. J Membr Biol, 2005. 204(1): p. 33-8.
Premkumar, A., L. Wilson, G.D. Ewart, and P.W. Gage, Cation-selective ion channels formed by p7 of hepatitis C virus are blocked by hexamethylene amiloride. FEBS Lett, 2004. 557(1-3): p. 99-103.
Wilson, L., P. Gage, and G. Ewart, Validation of coronavirus E proteins ion channels as targets for antiviral drugs. Adv Exp Med Biol, 2006. 581: p. 573-8.
Wilson, L., P. Gage, and G. Ewart, Hexamethylene amiloride blocks E protein ion channels and inhibits coronavirus replication. Virology, 2006. 353(2): p. 294-306.
Wilson, L., C. McKinlay, P. Gage, and G. Ewart, SARS coronavirus E protein forms cation-selective ion channels. Virology, 2004. 330(1): p. 322-31.
Sakai, A., M.S. Claire, K. Faulk, S. Govindarajan, S.U. Emerson, R.H. Purcell, and J. Bukh, The p7 polypeptide of hepatitis C virus is critical for infectivity and contains functionally important genotype-specific sequences. Proc Natl Acad Sci U S A, 2003. 100(20): p. 11646-51.
Griffin, S.D., L.P. Beales, D.S. Clarke, O. Worsfold, S.D. Evans, J. Jaeger, M.P. Harris, and D.J. Rowlands, The p7 protein of hepatitis C virus forms an ion channel that is blocked by the antiviral drug, Amantadine. FEBS Lett, 2003. 535(1-3): p. 34-8.
Pavlovic, D., D.C. Neville, O. Argaud, B. Blumberg, R.A. Dwek, W.B. Fischer, and N. Zitzmann, The hepatitis C virus p7 protein forms an ion channel that is inhibited by long-alkyl-chain iminosugar derivatives. Proc Natl Acad Sci U S A, 2003. 100(10): p. 6104-8.
Hay, A.J., A.J. Wolstenholme, J.J. Skehel, and M.H. Smith, The molecular basis of the specific anti-influenza action of amantadine. Embo J, 1985. 4(11): p. 3021-4. Duff, K.C. and R.H. Ashley, The transmembrane domain of influenza A M2 protein forms amantadine-sensitive proton channels in planar lipid bilayers. Virology, 1992. 190(1): p. 485-9.
De Clercq, E., Antiviral agents active against influenza A viruses. Nat Rev Drug Discov, 2006. 5(12): p. 1015-25.
Miller, C., Ion channel reconstitution. 1986, New York and London: Plenum Press. Buckwold, V.E., B.E. Beer, and R.O. Donis, Bovine viral diarrhea virus as a surrogate model of hepatitis C virus for the evaluation of antiviral agents. Antiviral Res, 2003. 60(1): p. 1-15.
Harada, T., N. Tautz, and H.J. Thiel, E2-p7 region of the bovine viral diarrhea virus polyprotein: processing and functional studies. J Virol, 2000. 74(20): p. 9498-506.
Griffin, S., D. Clarke, C. McCormick, D. Rowlands, and M. Harris, Signal peptide cleavage and internal targeting signals direct the hepatitis C virus p7 protein to distinct intracellular membranes. J Virol, 2005. 79(24): p. 15525-36.
Buckwold, V.E., J. Wei, M. Wenzel-Mathers, and J. Russell, Synergistic in vitro interactions between alpha interferon and Ribavirin against bovine viral diarrhea virus and yellow fever virus as surrogate models of hepatitis C virus replication. Antimicrob Agents Chemother, 2003. 47(7): p. 2293-8.
Haqshenas, G., X. Dong, G. Ewart, S. Bowden, and E.J. Gowans, A 2a/1b full-length p7 inter-genotypic chimeric genome of hepatitis C virus is infectious in vitro. Virology, 2007. 360(1): p. 17-26.
Buckwold, V.E., J. Wei, M. Wenzel-Mathers, and J. Russell. 2003. Synergistic in vitro interactions between alpha interferon and Ribavirin against bovine viral diarrhea virus and yellow fever virus as surrogate models of hepatitis C virus replication. Antimicrob. Agents Chemother. 47:2293-2298.
Buckwold, V.E., B.E. Beer, and R.O. Donis. 2003. Bovine viral diarrhea virus as a surrogate model of hepatitis C virus for the evaluation of antiviral agents. Antiviral Res. 60:1-15.

## Claims

1. A composition comprising:
(A) 5-(1-methylpyrazol-4-yl)2-naphthoylguanidine or (6-(1-methylpyrazol-4-yl)-2-nahpthoyl)guanidine, or a pharmaceutically acceptable salt thereof, and
(B) IFN α-2b,
wherein:
5-(1-methylpyrazol-4-yl)2-naphthoylguanidine has the structure 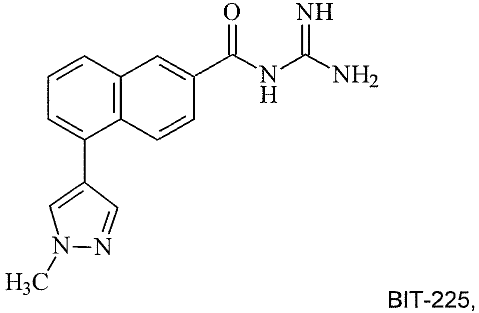 and
(6-(1-methylpyrazol-4-yl)-2-naphthoyl)guanidine has the structure

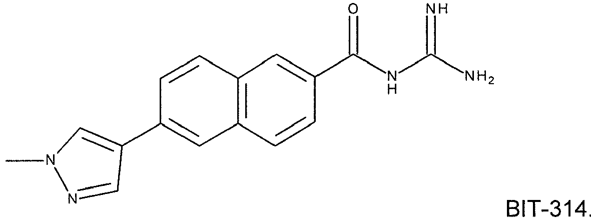

2. The composition according to claim 1, wherein the amine or imine group of the guanidyl portion of 5-(1-methylpyrazol-4-yl)2-naphthoylguanidine or (6-(1-methylpyrazol-4-yl)-2-nahpthoyl)guanidine is present as the free base, a hydrate, an organic or inorganic salt or a combination thereof.

3. The composition according to claim 1 or claim 2, wherein the composition further comprises Ribavirin.

4. The composition according to any one of claims 1 to 3, wherein the composition comprises:
5-(1-methylpyrazol-4-yl)2-naphthoylguanidine and IFN α-2b;
5-(1-methylpyrazol-4-yl)2-naphthoylguanidine and IFN α-2b and Ribavirin;
(6-(1-methylpyrazol-4-yl)-2-nahpthoyl)guanidine and IFN α-2b; or
(6-(1-methylpyrazol-4-yl)-2-naphthoyl)guanidine and IFN α-2b and Ribavirin.

5. A pharmaceutical composition for use in the treatment of HCV, comprising a composition according to any one of claims 1 to 4 together with one or more pharmaceutical acceptable carriers or excipients.

6. A composition comprising:
(A) 5-(1-methylpyrazol-4-yl)2-naphthoylguanidine or (6-(1-methylpyrazol-4-yl)-2-nahpthoyl)guanidine, or a pharmaceutically acceptable salt thereof, and
(B) IFN α-2b,
for use in the treatment of HCV infection as a combined preparation for simultaneous, separate or sequential use, in any order, wherein:
5-(1-methylpyrazol-4-yl)2-naphthoylguanidine has the structure 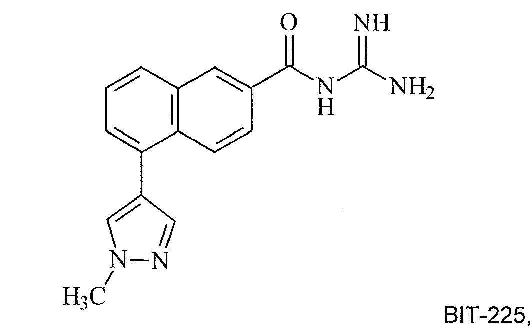 and
(6-(1-methylpyrazol-4-yl)-2-naphthoyl)guanidine has the structure

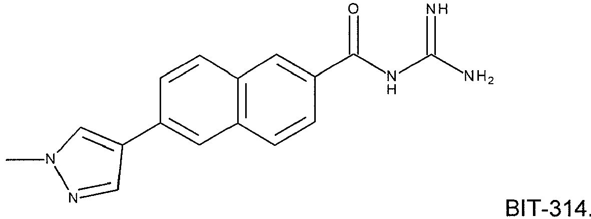

7. The composition for use according to claim 6, wherein the composition further comprises Ribavirin.

8. The composition for use according to claim 6 or claim 7, wherein the composition comprises:
5-(1-methylpyrazol-4-yl)2-naphthoylguanidine and IFN α-2b;
5-(1-methylpyrazol-4-yl)2-naphthoylguanidine and IFN α-2b and Ribavirin;
(6-(1-methylpyrazol-4-yl)-2-napthoyl)guanidine and IFN α-2b; or
(6-(1-methylpyrazol-4-yl)-2-napthoyl)guanidine and IFN α-2b and Ribavirin.

## Patentansprüche

1. Zusammensetzung, umfassend:
(A) 5-(1-Methylpyrazol-4-yl)2-Naphthoylguanidin oder (6-(1-Methylpyrazol-4-yl)-2-Naphthoyl)guanidin, oder ein pharmazeutisch unbedenkliches Salz davon, und
(B) IFN α-2b, wobei:
5-(1-Methylpyrazol-4-yl)2-Naphthoylguanidin die folgende Struktur hat 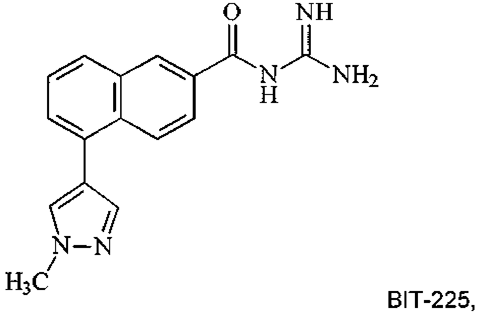 und
(6-(1-Methylpyrazol-4-yl)-2-Naphthoyl)guanidin die folgende Struktur hat

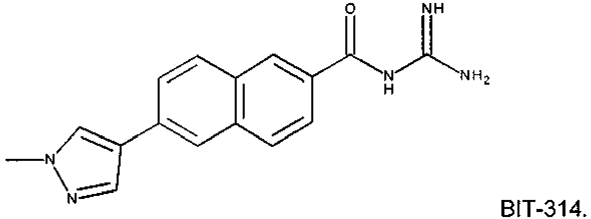

2. Die Zusammensetzung laut Patentanspruch 1, bei der die Amin- oder Imingruppe des Guanidyl-Abschnitts von 5-(1-Methylpyrazol-4-yl)2-Naphthoylguanidin oder (6-(1-Methylpyrazol-4-yl)-2- Naphthoyl)guanidin als die freie Base, ein Hydrat, ein organisches oder anorganisches Salz oder eine Kombination davon vorhanden ist.

3. Die Zusammensetzung nach Anspruch 1 oder Anspruch 2, wobei die Zusammensetzung außerdem Ribavirin enthält.

4. Die Zusammensetzung nach einem der Ansprüche 1 bis 3, wobei die Zusammensetzung Folgendes umfasst:
5-(1-Methylpyrazol-4-yl)2-Naphthoylguanidin und IFN α-2b;
5-(1-Methylpyrazol-4-yl)2-Naphthoylguanidin und IFN α-2b und Ribavirin;
(6-(1-Methylpyrazol-4-yl)-2-Naphthoyl)guanidin und IFN α-2b; oder
(6-(1-Methylpyrazol-4-yl)-2-Naphthoyl)guanidin und IFN α-2b und Ribavirin.

5. Eine pharmazeutische Zusammensetzung für den Einsatz in der Behandlung von HCV, die eine Zusammensetzung nach einem der Ansprüche 1 bis 4 gemeinsam mit einem oder mehr pharmazeutisch unbedenklichen Trägern oder Hilfsstoffen enthält.

6. Zusammensetzung, umfassend:
(A) 5-(1-Methylpyrazol-4-yl)2-Naphthoylguanidin oder (6-(1-Methylpyrazol-4-yl)-2-Naphthoyl)guanidin, oder ein pharmazeutisch unbedenkliches Salz davon, und
(B) IFN α-2b,
für den Einsatz in der Behandlung von HCV-Infektionen als eine kombinierte Zubereitung für eine gleichzeitige, separate oder aufeinanderfolgende Verwendung, in einer beliebigen Reihenfolge, wobei:
5-(1-Methylpyrazol-4-yl)2-Naphthoylguanidin die folgende Struktur hat 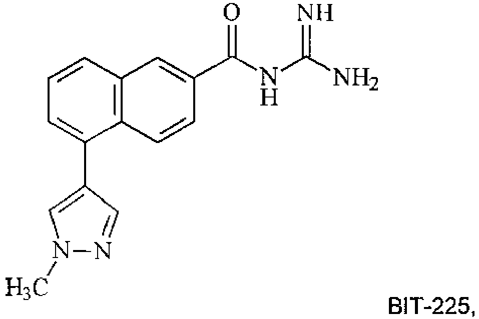 und
(6-(1-Methylpyrazol-4-yl)-2-Naphthoyl)guanidin die folgende Struktur hat

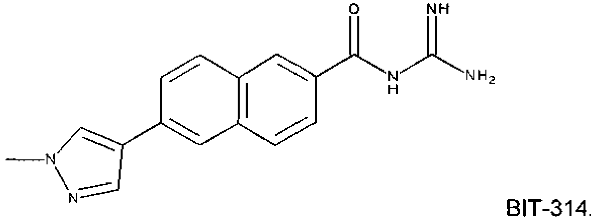

7. Die Zusammensetzung zur Verwendung nach Anspruch 6, wobei die Zusammensetzung außerdem Ribavirin enthält.

8. Die Zusammensetzung zur Verwendung nach Anspruch 6 oder Anspruch 7, wobei die Zusammensetzung Folgendes umfasst:
5-(1-Methylpyrazol-4-yl)2-Naphthoylguanidin und IFN α-2b;
5-(1-Methylpyrazol-4-yl)2-Naphthoylguanidin und IFN α-2b und Ribavirin;
(6-(1-Methylpyrazol-4-yl)-2-Naphthoyl)guanidin und IFN α-2b; oder
(6-(1-Methylpyrazol-4-yl)-2-Naphthoyl)guanidin und IFN α-2b und Ribavirin.

## Revendications

1. Composition comprenant :
(A) 5-(1-méthylpyrazol-4-yl)2-naphthoylguanidine ou (6-(1-méthylpyrazol-4-yle)-2-nahpthoyle) guanidine, ou un sel pharmaceutiquement acceptable de ceux-ci, et
(B) IFN α-2b, où :
5-(1-méthylpyrazol-4-yle)2-naphthoylguanidine présente la structure 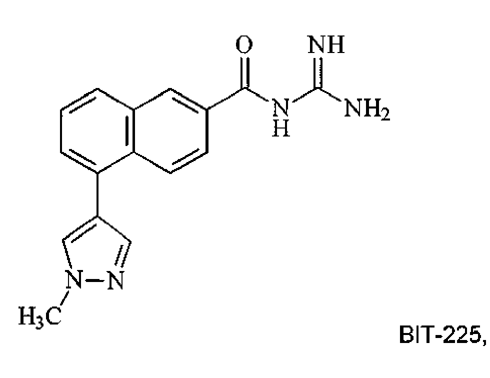 et
6-(1-méthylpyrazol-4-yle)-2-naphthoyle)guanidine présente la structure

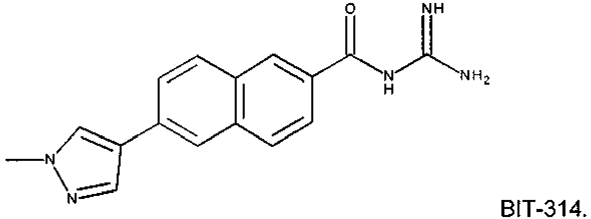

2. Composition selon la revendication 1, où l'amine ou le groupe imine de la partie guanidyle de 5-(1-méthylpyrazol-4-yle)2-naphthoylguanidine ou (6-(1-méthylpyrazol-4-yle)-2-nahpthoyle)guanidine est présente sous forme de base libre, d'un hydrate, d'un sel inorganique ou organique ou d'une combinaison de ceux-ci.

3. Composition selon la revendication 1 ou la revendication 2, dans laquelle la composition pharmaceutique comprend en outre du Ribavirine.

4. Composition selon l'une quelconque des revendications 1 à 3, où la composition comprend :
5-(1-méthylpyrazol-4-yle)2-naphthoylguanidine et IFN α-2b ;
5-(1-méthylpyrazol-4-yle)2-naphthoylguanidine et IFN α-2b et Ribavirine ;
(6-(1-méthylpyrazol-4-yle)-2-naphthoyle)guanidine et IFN α-2b ; ou
(6-(1-méthylpyrazol-4-yle)-2-naphthoyle)guanidine et IFN α-2b et Ribavirine.

5. Composition pharmaceutique pour utilisation dans le traitement de l'hépatite C, comprenant une composition selon l'une des revendications 1 à 4 en conjonction avec un ou plusieurs vecteurs ou excipients pharmaceutiquement acceptables.

6. Composition comprenant :
(A) 5-(1-méthylpyrazol-4-yle)2-naphthoylguanidine ou (6-(1-méthylpyrazol-4-yle)-2-nahpthoyle)guanidine, ou un sel pharmaceutiquement acceptable de ceux-ci, et
(B) IFN α-2b,
pour utilisation dans le traitement de l'infection par l'hépatite C comme préparation combinée pour une utilisation simultanée, séparée ou séquentielle, dans n'importe quel ordre, où :
5-(1-méthylpyrazol-4-yle)2-naphthoylguanidine présente la structure 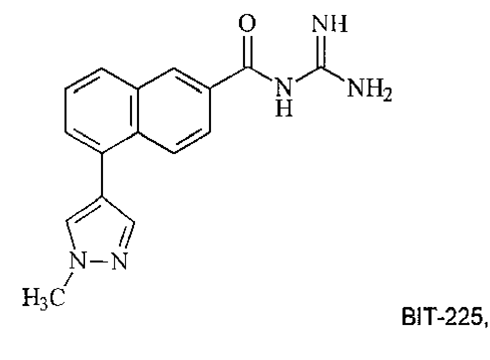 et
(6-(1-méthylpyrazol-4-yle)-2-naphthoyle)guanidine présente la structure

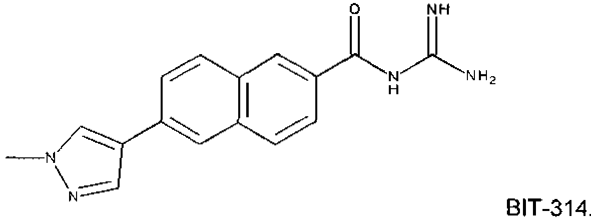

7. Composition pour utilisation selon la revendication 6, où la composition comprend en outre la Ribavirine.

8. Composition pour utilisation selon la revendication 6 ou selon la revendication 7, où la composition comprend :
5-(1-méthylpyrazol-4-yle)2-naphthoylguanidine et IFN α-2b ;
5-(1-méthylpyrazol-4-yle)2-naphthoylguanidine et IFN α-2b et Ribavirine ;
(6-(1-méthylpyrazol-4-yle)-2-naphthoyle)guanidine et IFN α-2b ; ou
(6-(1-méthylpyrazol-4-yle)-2-naphthoyle)guanidine et IFN α-2b et Ribavirine.
